# EUROPEAN PATENT APPLICATION

(11) **EP 1 275 659 A1**
(43) Date of publication of application: **15.01.2003**
(21) Application number: 01921804.9
(22) Date of filing: 11.04.2001
(51) Int. Cl.: C07K 14/47, C12N 15/12, C07K 16/18, A61K 39/395, A61K 38/17, A61K 45/00, A61P 25/28, A61P 3/04, A61P 15/00, A61P 13/12, A61P 13/10, A61P 15/08, A61P 15/16, A61P 21/00

(54) **NOVEL PHYSIOLOGICALLY ACTIVE PEPTIDES AND USE THEREOF**

(30) Priority: 11.04.2000 JP 2000109382
(71) Applicant: Takeda Chemical Industries, Ltd., Osaka-shi, Osaka 541-0045 (JP)
(72) Inventor: OHTAKI, Tetsuya, Tsukuba-shi, Ibaraki 305-0821 (JP); KUMANO, Satoshi;, Tsukuba-shi, Ibaraki 305-0035 (JP)
(74) Representative: Lewin, John Harvey
(86) International application number: JP0103112
(87) International publication number: WO01077166

(57) **Abstract**

Peptides having the amino acid sequence represented by SEQ ID NO: 1 or a substantially the same amino acid sequence and being capable of binding to receptor proteins having the amino acid sequences represented by SEQ ID NO: 11, 12 or 13 or a substantially the same amino acid sequence, and precursors, amides, esters and salts of these peptides. DNAs, etc. encoding these peptides are usable in: (i) developing receptor-binding assay systems and screening candidate compounds for drugs with the use of an expression system of a recombinant receptor protein; (ii) developing drugs with little side effect such as memory function improving agents, appetite improving agents and function controllers for uterus, kidney, prostate, testis or skeletal muscle, etc.

Sequence Listing
<110> Takeda Chemical Industries, Ltd.
<120> Novel Physiological Active Peptide and its Use
<130> 662517
<160> 19

<210> 1
<211> 60
<212> PRT
<213> Mouse
<400> 1
Ala Pro Ala His Arg Gly Arg Gly Gly Trp Thr Leu Asn Ser Ala Gly
1               5                   10                  15
Tyr Leu Leu Gly Pro Val Leu Pro Val Ser Ser Lys Ala Asp Gln Gly
            20                  25                  30
Arg Lys Arg Asp Ser Ala Leu Glu Ile Leu Asp Leu Trp Lys Ile Ile
        35                  40                  45
Asp Gly Leu Pro Tyr Ser His Ser Pro Arg Met Thr
    50                  55                  60

<210> 2
<211> 117
<212> PRT
<213> Mouse
<400> 2
Met Ala Cys Ser Val His Leu Val Leu Phe Leu Thr Ile Leu Leu Ser
1               5                   10                  15
Leu Ala Glu Thr Pro Glu Ser Ala Pro Ala His Arg Gly Arg Gly Gly
            20                  25                  30

Trp Thr Leu Asn Ser Ala Gly Tyr Leu Leu Gly Pro Val Leu Pro Val
        35                  40                  45
Ser Ser Lys Ala Asp Gln Gly Arg Lys Arg Asp Ser Ala Leu Glu Ile
    50                  55                  60
Leu Asp Leu Trp Lys Ile Ile Asp Gly Leu Pro Tyr Ser His Ser Pro
65                  70                  75                  80
Arg Met Thr Lys Arg Thr Met Gly Glu Thr Phe Val lys Ala Asn Thr
                85                  90                  95
Gly Asp Met His Ile Leu Asp Lys Asn Val Pro Lys Glu Glu Ala Thr
            100                 105                 110
Leu Asp Ser Glu Ser
        115

<210> 3
<211> 180
<212> DNA
<213> Mouse
<400> 3
gcacctgctc acaggggacg aggaggctgg accctcaata gtgctggtta cctcctgggt 60
cctgtcctcc ccgtttcctc caaggccgac cagggcagga agagagactc agctcttgag 120
atcctagacc tgtggaagat cattgatgga cttccttatt cccactctcc aaggatgacc 180

<210> 4
<211> 28
<212> DNA
<213> Artificial Sequence
<220>
<223> Designed oligonucleotide Primer to amplify mouse GALR2 ligand
<400> 4

aggctggacc ctcaatagtg ctggttac     28

<210> 5
<211> 28
<212> DNA
<213> Artificial Sequence
<220>
<223> Designed oligonucleotide Primer to amplify mouse GALR2 ligand
<400> 5
ccatctatgg ccttccacag gtctagga     28

<210> 6
<211> 126
<212> DNA
<213> Mouse
<400> 6
aggctggacc ctcaatagtg ctggttacct cctgggtcct gtcctccccg tttcctccaa  60
ggccgaccag ggcaggaaga gagactcagc tcttgagatc ctagacctgt ggaaggccat 120
agatgg                                                             126

<210> 7
<211> 28
<212> DNA
<213> Artificial Sequence
<220>
<223> Designed oligonucleotide Primer for 5' RACE to amplify mouse GALR2
ligand
<400> 7
tccaaggccg accagggcag gaagagag     28

<210> 8

<211> 28

<212> DNA

<213> Artificial Sequence

<220>

<223> Designed oligonucleotide Primer for 3' RACE to amplify mouse GALR2

ligand

<400> 8

ggtctaggat ctcaagagct gagtctct     28

<210> 9

<211> 28

<212> DNA

<213> Artificial Sequence

<220>

<223> Designed oligonucleotide Primer to amplify mouse GALR2 ligand

<400> 9

gaaagacagt ctcaaatcaa ggacctgc     28

<210> 10

<211> 28

<212> DNA

<213> Artificial Sequence

<220>

<223> Designed oligonucleotide Primer to amplify mouse GALR2 ligand

<400> 10

tgagctggaa acgaaggagg agtgaggt     28

<210> 11

<211> 346

<212> PRT

<213> Rat

<400> 11

Met Glu Leu Ala Pro Val Asn Leu Ser Glu Gly Asn Gly Ser Asp Pro

1               5                   10                  15

Glu Pro Pro Ala Glu Pro Arg Pro Leu Phe Gly Ile Gly Val Glu Asn

            20                  25                  30

Phe Ile Thr Leu Val Val Phe Gly Leu Ile Phe Ala Met Gly Val Leu

        35                  40                  45

Gly Asn Ser Leu Val Ile Thr Val Leu Ala Arg Ser Lys Pro Gly Lys

    50                  55                  60

Pro Arg Ser Thr Thr Asn Leu Phe Ile Leu Asn Leu Ser Ile Ala Asp

65                  70                  75                  80

Leu Ala Tyr Leu Leu Phe Cys Ile Pro Phe Gln Ala Thr Val Tyr Ala

                85                  90                  95

Leu Pro Thr Trp Val Leu Gly Ala Phe Ile Cys Lys Phe Ile His Tyr

            100                 105                 110

Phe Phe Thr Val Ser Met Leu Val Ser Ile Phe Thr Leu Ala Ala Met

        115                 120                 125

Ser Val Asp Arg Tyr Val Ala Ile Val His Ser Arg Arg Ser Ser Ser

    130                 135                 140

Leu Arg Val Ser Arg Asn Ala Leu Leu Gly Val Gly Phe Ile Trp Ala

145                 150                 155                 160

Leu Ser Ile Ala Met Ala Ser Pro Val Ala Tyr Tyr Gln Arg Leu Phe

                165                 170                 175

His Arg Asp Ser Asn Gln Thr Phe Cys Trp Glu His Trp Pro Asn Gln

            180                 185                 190

Leu His Lys Lys Ala Tyr Val Val Cys Thr Phe Val Phe Gly Tyr Leu
        195                 200                 205
Leu Pro Leu Leu Leu Ile Cys Phe Cys Tyr Ala Lys Val Leu Asn His
    210                 215                 220
Leu His Lys Lys Leu Lys Asn Met Ser Lys Lys Ser Glu Ala Ser Lys
225                 230                 235                 240
Lys Lys Thr Ala Gln Thr Val Leu Val Val Val Val Val Phe Gly Ile
                245                 250                 255
Ser Trp Leu Pro His His Val Ile His Leu Trp Ala Glu Phe Gly Ala
            260                 265                 270
Phe Pro Leu Thr Pro Ala Ser Phe Phe Phe Arg Ile Thr Ala His Cys
        275                 280                 285
Leu Ala Tyr Ser Asn Ser Ser Val Asn Pro Ile Ile Tyr Ala Phe Leu
    290                 295                 300
Ser Glu Asn Phe Arg Lys Ala Tyr Lys Gln Val Phe Lys Cys Arg Val
305                 310                 315                 320
Cys Asn Glu Ser Pro His Gly Asp Ala Lys Glu Lys Asn Arg Ile Asp
                325                 330                 335
Thr Pro Pro Ser Thr Asn Cys Thr His Val
            340                 345

<210> 12
<211> 372
<212> PRT
<213> Rat
<400> 12
Met Asn Gly Ser Gly Ser Gln Gly Ala Glu Asn Thr Ser Gln Glu Gly
1               5                   10                  15
Gly Ser Gly Gly Trp Gln Pro Glu Ala Val Leu Val Pro Leu Phe Phe

            20                  25                  30
Ala Leu Ile Phe Leu Val Gly Thr Val Gly Asn Ala Leu Val Leu Ala
        35                  40                  45
Val Leu Leu Arg Gly Gly Gln Ala Val Ser Thr Thr Asn Leu Phe Ile
    50                  55                  60
Leu Asn Leu Gly Val Ala Asp Leu Cys Phe Ile Leu Cys Cys Val Pro
65                  70                  75                  80
Phe Gln Ala Thr Ile Tyr Thr Leu Asp Asp Trp Val Phe Gly Ser Leu
                85                  90                  95
Leu Cys Lys Ala Val His Phe Leu Ile Phe Leu Thr Met His Ala Ser
            100                 105                 110
Ser Phe Thr Leu Ala Ala Val Ser Leu Asp Arg Tyr Leu Ala Ile Arg
        115                 120                 125
Tyr Pro Leu His Ser Arg Glu Leu Arg Thr Pro Arg Asn Ala Leu Ala
    130                 135                 140
Ala Ile Gly Leu Ile Trp Gly Leu Ala Leu Leu Phe Ser Gly Pro Tyr
145                 150                 155                 160
Leu Ser Tyr Tyr Arg Gln Ser Gln Leu Ala Asn Leu Thr Val Cys His
                165                 170                 175
Pro Ala Trp Ser Ala Pro Arg Arg Arg Ala Met Asp Leu Cys Thr Phe
            180                 185                 190
Val Phe Ser Tyr Leu Leu Pro Val Leu Val Leu Ser Leu Thr Tyr Ala
        195                 200                 205
Arg Thr Leu Arg Tyr Leu Trp Arg Thr Val Asp Pro Val Thr Ala Gly
    210                 215                 220
Ser Gly Ser Gln Arg Ala Lys Arg Lys Val Thr Arg Met Ile Ile Ile
225                 230                 235                 240
Val Ala Val Leu Phe Cys Leu Cys Trp Met Pro His His Ala Leu Ile
                245                 250                 255

Leu Cys Val Trp Phe Gly Arg Phe Pro Leu Thr Arg Ala Thr Tyr Ala
            260                 265                 270
Leu Arg Ile Leu Ser His Leu Val Ser Tyr Ala Asn Ser Cys Val Asn
        275                 280                 285
Pro Ile Val Tyr Ala Leu Val Ser Lys His Phe Arg Lys Gly Phe Arg
    290                 295                 300
Lys Ile Cys Ala Gly Leu Leu Arg Pro Ala Pro Arg Arg Ala Ser Gly
305                 310                 315                 320
Arg Val Ser Ile Leu Ala Pro Gly Asn His Ser Gly Ser Met Leu Glu
                325                 330                 335
Gln Glu Ser Thr Asp Leu Thr Gln Val Ser Glu Ala Ala Gly Pro Leu
            340                 345                 350
Val Pro Pro Pro Ala Leu Pro Asn Cys Thr Ala Ser Ser Arg Thr Leu
        355                 360                 365
Asp Pro Ala Cys
    370

<210> 13
<211> 370
<212> PRT
<213> Rat
<400> 13
Met Ala Asp Ile Gln Asn Ile Ser Leu Asp Ser Pro Gly Ser Val Gly
1               5                   10                  15
Ala Val Ala Val Pro Val Ile Phe Ala Leu Ile Phe Leu Leu Gly Met
            20                  25                  30
Val Gly Asn Gly Leu Val Leu Ala Val Leu Leu Gln Pro Gly Pro Ser
        35                  40                  45
Ala Trp Gln Glu Pro Ser Ser Thr Thr Asp Leu Phe Ile Leu Asn Leu

    50                  55                  60
Ala Val Ala Asp Leu Cys Phe Ile Leu Cys Cys Val Pro Phe Gln Ala
65                  70                  75                  80
Ala Ile Tyr Thr Leu Asp Ala Trp Leu Phe Gly Ala Phe Val Cys Lys
                85                  90                  95
Thr Val His Leu Leu Ile Tyr Leu Thr Met Tyr Ala Ser Ser Phe Thr
            100                 105                 110
Leu Ala Ala Val Ser Leu Asp Arg Tyr Leu Ala Val Arg His Pro Leu
        115                 120                 125
Arg Ser Arg Ala Leu Arg Thr Pro Arg Asn Ala Arg Ala Ala Val Gly
    130                 135                 140
Leu Val Trp Leu Leu Ala Ala Leu Phe Ser Ala Pro Tyr Leu Ser Tyr
145                 150                 155                 160
Tyr Gly Thr Val Arg Tyr Gly Ala Leu Glu Leu Cys Val Pro Ala Trp
                165                 170                 175
Glu Asp Ala Arg Arg Arg Ala Leu Asp Val Ala Thr Phe Ala Ala Gly
            180                 185                 190
Tyr Leu Leu Pro Val Ala Val Val Ser Leu Ala Tyr Gly Arg Thr Leu
        195                 200                 205
Cys Phe Leu Trp Ala Ala Val Gly Pro Ala Gly Ala Ala Ala Ala Glu
    210                 215                 220
Ala Arg Arg Arg Ala Thr Gly Arg Ala Gly Arg Ala Met Leu Ala Val
225                 230                 235                 240
Ala Ala Leu Tyr Ala Leu Cys Trp Gly Pro His His Ala Leu Ile Leu
                245                 250                 255
Cys Phe Trp Tyr Gly Arg Phe Ala Phe Ser Pro Ala Thr Tyr Ala Cys
            260                 265                 270
Arg Leu Ala Ser His Cys Leu Ala Tyr Ala Asn Ser Cys Leu Asn Pro
        275                 280                 285

Leu Val Tyr Ser Leu Ala Ser Arg His Phe Arg Ala Arg Phe Arg Arg
    290                 295                 300
Leu Trp Pro Cys Gly Arg Arg Arg His Arg His His His Arg Ala His
305                 310                 315                 320
Arg Ala Leu Arg Arg Val Gln Pro Ala Ser Ser Gly Pro Ala Gly Tyr
                325                 330                 335
Pro Gly Asp Ala Arg Pro Arg Gly Trp Ser Met Glu Pro Arg Gly Asp
            340                 345                 350
Ala Leu Arg Gly Gly Gly Glu Thr Arg Leu Thr Leu Ser Pro Arg Gly
        355                 360                 365
Pro Gln
    370

<210> 14
<211> 60
<212> PRT
<213> Porcine
<400> 14
Ala Pro Val His Arg Gly Arg Gly Gly Trp Thr Leu Asn Ser Ala Gly
1               5                   10                  15
Tyr Leu Leu Gly Pro Val Leu His Pro Pro Ser Arg Ala Glu Gly Gly
            20                  25                  30
Gly Lys Gly Lys Thr Ala Leu Gly Ile Leu Asp Leu Trp Lys Ala Ile
        35                  40                  45
Asp Gly Leu Pro Tyr Pro Gln Ser Gln Leu Ala Ser
    50                  55                  60

<210> 15
<211> 60

<212> PRT

<213> Rat

<400> 15

Ala Pro Ala His Arg Gly Arg Gly Gly Trp Thr Leu Asn Ser Ala Gly

1               5                   10                  15

Tyr Leu Leu Gly Pro Val Leu His Leu Ser Ser Lys Ala Asn Gln Gly

            20                  25                  30

Arg Lys Thr Asp Ser Ala Leu Glu Ile Leu Asp Leu Trp Lys Ala Ile

        35                  40                  45

Asp Gly Leu Pro Tyr Ser Arg Ser Pro Arg Met Thr

    50                  55                  60

<210> 16

<211> 60

<212> PRT

<213> Human

<400> 16

Ala Pro Ala His Arg Gly Arg Gly Gly Trp Thr Leu Asn Ser Ala Gly

1               5                   10                  15

Tyr Leu Leu Gly Pro Val Leu His Leu Pro Gln Met Gly Asp Gln Asp

            20                  25                  30

Gly Lys Arg Glu Thr Ala Leu Glu Ile Leu Asp Leu Trp Lys Ala Ile

        35                  40                  45

Asp Gly Leu Pro Tyr Ser His Pro Pro Gln Pro Ser

    50                  55                  60

<210> 17

<211> 1041

<212> DNA

<213> Rat

<400> 17

atggaactgg ctccggtgaa cctcagtgaa gggaatggga gcgaccctga acctccagcg 60

gaacccaggc cgctcttcgg catcggcgtg gagaacttca tcacgctggt ggtgtttggc 120

cttattttcg cgatgggcgt gctgggcaac agcctggtga tcaccgtgct ggcgcgcagc 180

aaaccgggca agccgcgcag caccaccaac ctgttcatcc tcaacctgag catcgcagac 240

ctggcctacc tgctcttctg catccctttc caggccaccg tgtacgcact gcccacctgg 300

gtgctgggcg ccttcatctg caagtttata cactacttct tcaccgtgtc catgctcgtg 360

agcatcttca ccctggccgc gatgtctgtg gatcgctatg tggccattgt gcattcacgg 420

cgctcctcct ccctcagggt gtcccgcaac gcgctgctgg gcgtgggctt catctgggcg 480

ctgtccatcg ctatggcctc gccggtggcc tactaccagc gcctttttca tcgggacagc 540

aaccaaacct tctgctggga gcactggccc aaccaactcc acaagaaggc ttacgtggtg 600

tgcactttcg tctttggtta ccttctgccc ttactgctca tctgcttttg ctatgccaag 660

gttctcaatc atctgcataa aaagttgaag aacatgtcaa aaaagtcaga ggcatccaag 720

aaaaagactg cacagactgt cctggtggtc gttgtggtat ttggcatatc atggctgccc 780

catcatgtca tccacctctg ggctgagttc ggagcattcc cgctgacccc agcttccttc 840

ttcttcagaa tcactgccca ctgcctggca tacagcaact cctcggtgaa ccccatcatc 900

tacgcctttc tctcagaaaa cttccggaag gcgtacaagc aagtgttcaa gtgccgtgtt 960

tgcaatgagt cgccgcacgg cgatgctaaa gaaaagaacc gaatagatac cccgccctcc 1020

accaactgca cccacgtgtg a 1041

<210> 18

<211> 1119

<212> DNA

<213> Rat

<400> 18

atgaatggct ccggcagcca gggcgcggag aacacgagcc aggaaggcgg tagcggcggc 60

tggcagcctg aggcggtcct tgtaccccta tttttcgcgc tcatcttcct cgtgggcacc 120

gtgggcaacg cgctggtgct ggcggtgctg ctgcgcggcg gccaggcggt cagcaccacc 180

aacctgttca tcctcaacct gggcgtggcc gacctgtgtt tcatcctgtg ctgcgtgcct 240
ttccaggcca ccatctacac cctggacgac tgggtgttcg gctcgctgct ctgcaaggct 300
gttcatttcc tcatctttct cactatgcac gccagcagct tcacgctggc cgccgtctcc 360
ctggacaggt atctggccat ccgctacccg ctgcactccc gagagttgcg cacacctcga 420
aacgcgctgg ccgccatcgg gctcatctgg gggctagcac tgctcttctc cgggccctac 480
ctgagctact accgtcagtc gcagctggcc aacctgacag tatgccaccc agcatggagc 540
gcacctcgac gtcgagccat ggacctctgc accttcgtct ttagctacct gctgccagtg 600
ctagtcctca gtctgaccta tgcgcgtacc ctgcgctacc tctggcgcac agtcgacccg 660
gtgactgcag gctcaggttc ccagcgcgcc aaacgcaagg tgacacggat gatcatcatc 720
gtggcggtgc ttttctgcct ctgttggatg ccccaccacg cgcttatcct ctgcgtgtgg 780
tttggtcgct tcccgctcac gcgtgccact tacgcgttgc gcatcctttc acacctagtt 840
tcctatgcca actcctgtgt caaccccatc gtttacgctc tggtctccaa gcatttccgt 900
aaaggtttcc gcaaaatctg cgcgggcctg ctgcgccctg ccccgaggcg agcttcgggc 960
cgagtgagca tcctggcgcc tgggaaccat agtggcagca tgctggaaca ggaatccaca 1020
gacctgacac aggtgagcga ggcagccggg ccccttgtcc caccacccgc acttcccaac 1080
tgcacagcct cgagtagaac cctggatccg gcttgttaa 1119

<210> 19
<211> 1113
<212> DNA
<213> Rat
<400> 19
atggctgaca tccagaacat ttcgctggac agcccaggga gcgtaggggc tgtggcagtg 60
cctgtgatct ttgccctcat cttcctgttg ggcatggtgg gcaatgggct ggtgttggct 120
gtgctactgc agcctggccc aagtgcctgg caggagccaa gcagtaccac agatctcttc 180
atcctcaact tggccgtggc cgacctttgc ttcatcctgt gctgcgtgcc cttccaggca 240
gccatctaca cactggatgc ctggctcttt ggggctttcg tgtgcaagac ggtacatctg 300
ctcatctacc tcaccatgta tgccagcagc ttcaccctgg cggccgtctc cctggacagg 360
tacctggctg tgcggcaccc actgcgctcc agagccctgc gcaccccgcg caacgcgcgc 420

gccgccgtgg ggctcgtgtg gctgctggcg gctctctttt ccgcgcccta cctaagctat 480

tacggcacgg tgcgctacgg cgcgctcgag ctctgcgtgc ccgcttggga ggacgcgcgg 540

cggcgcgcgc tggacgtggc caccttcgcc gcgggctacc tgctgccggt ggccgtggtg 600

agcctggcct acggacgcac gctatgtttc ctatgggccg ccgtgggtcc cgcgggcgcg 660

gcggcagcag aggcgcgcag acgggcgacc ggccgggcgg gacgcgccat gctggcagtg 720

gccgcgctct acgcgctttg ctggggcccg caccacgcgc tcatcctctg cttctggtac 780

ggccgcttcg ccttcagccc ggccacctac gcctgtcgcc tggcctcgca ctgcctcgcc 840

tacgccaact cctgccttaa cccgctcgtc tactcgctcg cctcgcgcca cttccgcgcg 900

cgcttccgcc gcctgtggcc ctgcggccgt cgccgccacc gccaccacca ccgcgctcat 960

cgagccctcc gtcgtgtcca gccggcgtct tcgggccccg ccggttatcc cggcgacgcc 1020

aggcctcgtg gttggagtat ggagcccaga ggggatgctc tgcgtggtgg tggagagact 1080

agactaaccc tgtcccccag gggacctcaa taa 1113

<210> 20

<211> 351

<212> DNA

<213> Rat

<400> 20

atggcctgct ccgtacatct ggtcctcttc ctcaccatct tgctgagcct ggcagaaaca 60

ccggaatctg cacctgctca caggggacga ggaggctgga ccctcaatag tgctggttac 120

ctcctgggtc ctgtcctccc cgtttcctcc aaggccgacc agggcaggaa gagagactca 180

gctcttgaga tcctagacct gtggaagatc attgatggac ttccttattc ccactctcca 240

aggatgacca aaaggacaat gggagaaacg tttgtcaaag cgaatactgg agatatgcac 300

atactggaca agaatgttcc caaggaagaa gccaccctgg actcagagag t 351

## Description

### FIELD OF THE INVENTION

The present invention relates to an activating factor (a ligand peptide and the like) for a galanin receptor.

### BACKGROUND ART

Galanin is a physiologically active peptide comprising 29 amino acid residues that has been first found in an extract from swine small intestine (FEBS Lett., 164, pp124-128 (1983)), and has been found thereafter in various mammals, birds, reptiles and fishes besides swine. The amino acid sequences of galanin have been reported in human (FEBS Lett., 283, pp189-194 (1991)), bovine (FEBS Lett., 234, pp400-406 (1988)), rat (J. Biol. Chem., 262, pp16755-16758 (1987)), and sheep (Peptides, 12, pp855-859 (1991)). These sequences are conserved among species in 15 amino acid residues from the N-terminal.

A precursor protein comprising 123 amino acid residues [preprogalanin (1-123); Proc. Natl. Acad. Sci. USA, 83, pp6287-6291 (1986)], a precursor having N-terminal residues longer than that in galanin by 9 amino acids [preprogalanin (24-61) amide], and a precursor deficient of four N-terminal residues of galanin [preprogalanin (37-61) amide] have been known in the swine galanin (Peptides, 13, pp1055 1060 (1992)) are known in swine galanin.

Physiological actions of galanin known in the art include acetylcholine release inhibition at hippocampus (Brain Research, 709, pp81-87 (1996)), feeding center enhancing action at hypothalamus (Obesity Research, 3, pp5735-5895 (1995)), pituitary gland hormone release enhancing action at pituitary gland (Neuroscience Letter, 75, pp49-54 (1987); Endocrinology, 134, pp529-536 (1994); Peptides, 7, pp51-53 (1986)) and insulin secretion inhibitory action at pancreas (Acta Physiol. Scand., 139, pp591-596 (1990)). These physiological actions are considered to be exerted by being mediated via the galanin receptors.

Three kinds of subtypes (GALR1, GALR2, GALR3) are found in the galanin receptors, wherein the gene of GALR1 is cloned in human, rat and mouse (Proc. Natl. Acad. Sci. USA, 90, pp3845-3849 (1993); J. Mol. Neurosci., 6, pp33-41 (1955); FEBS Lett., 411, pp225-230 (1997)), the gene of GALR2 is cloned in rat (FEBS Lett., 405, pp285-290 (1997); Mol. Pharmacol., 52, pp337-343 (1997); J. Biol. Chem., 272, pp24612-24616 (1997)), and the gene of GALR3 is cloned in rat (J. Biol. Chem., 272, pp31949-31952 (1997)). These three kind of galanin receptors involve seven hydrophobic regions (membrane penetrating domains) characteristic of G-protein conjugation type receptor, and are considered to stimulate intracellular information transmittance system by activating the G-protein.

It has been confirmed that galanin binds to any of these three kind of galanin receptor subtypes. Binding affinity of galanin is strongest for GALR1, and decreases in the order of GALR2 and GALR3. Affinity of galanin to GALR3 is about ten times weaker than that of galanin to GALR1 (J. Biol. Chem., 272, 31949-31952, 1997). It has been also reported that galanin provokes inhibition of cAMP production in the GALR1 expression cells (Proc. Natl. Acad. Sci. USA, 90, 3845-3849, 1993), and inhibition of cAMP production (Mol. Pharmacol., 52, pp337-343 (1997)), hypermetabolism of inositol-phosphate metabolic system and increment of intracellular calcium concentration (J. Biol. Chem., 272, 24612-24616, 1997) in the GALR2 expression cells.

### Objects of the Invention

Substances that inhibit binding between the G-protein conjugated receptor and physiologically active substances (i.e. ligands), and substances which induce signal transduction similar to that of the physiologically active substances (i.e. ligands) by binding to the receptor have been utilized as medicines that can control biological functions as antagonists or agonists specific to these receptors. Accordingly, it would be a crucial means for finding the agonists and antagonists to find a novel G-protein conjugated receptor protein that is important in manifesting physiological functions in vivo and may serve as a target of development of medicines, and to find a ligand specific to the novel G-protein conjugated receptor protein.

At the same time, in order to elucidate physiological mechanisms of receptor, it is also important for creating effective medicines to obtain a counterpart gene of, for example, mouse against the human receptor gene or ligand gene, to inquire protein chemical properties and biological activities of the gene product, and to presume its functions in human through detailed investigation of quantitative and qualitative kinetics and physiological mechanisms of the product in vivo.

Furthermore, it is essential for creating medicines to select and determine candidate compounds in consideration of differences among species in case of selecting the antagonist and agonist as candidates.

As described above, the present invention provides a mouse homologue ligand (hereinafter, sometimes referred to as "a peptide according to the present invention") of a ligand derived from human, specific to a useful galanin receptor (WO 99/48920).

### Summary of the Invention

The present inventors have assembled galanin receptor GALR2 expression cells and galanin receptor GALR1 expression cells, and developed a simple assay method for measuring an agonist activity against each subtype of the galanin receptors, that is a [³⁵S]GTPγS binding test method by using these cells. As a result of screening of the GALR2 agonist using the assay method, the present inventors have successfully found a novel activating factor derived from mouse, which has a different activation mechanism against each subtype of the galanin receptor. The inventors have also found on the basis of the discovery described above that it is possible to screen a compound that can modify the binding affinity between the novel activating factor and galanin receptor.

That is, the present invention relates to:
(1) a peptide comprising the same or substantially the same amino acid sequence as that represented by SEQ ID NO: 1, or a precursor, an amide, an ester or a salt thereof;
(2) a precursor according to (1) comprising the same or substantially the same amino acid sequence as that represented by SEQ ID NO: 2;
(3) a DNA comprising the DNA encoding the peptide or precursor thereof according to (1);
(4) a DNA according to (3) comprising a nucleotide sequence represented by SEQ ID NO: 3 or SEQ ID NO:20;
(5) a recombinant vector comprising the DNA according to (3);
(6) a transformant transformed by the recombinant vector according to (5);
(7) a method for producing the peptide or a precursor, an amide, an ester or a salt thereof according to (1), comprising cultivating the transformant according to (6) to produce and accumulate the peptide or a precursor, an amide, an ester or a salt thereof according to (1);
(8) an antibody against the peptide or precursor thereof according to (1);
(9) a medicine comprising the antibody according to (8);
(10) a diagnostics comprising the antibody according to (8);
(11) a medicine comprising the peptide or a precursor, an amide, an ester or a salt thereof according to (1);
(12) a medicine according to (11) which is a memory function improving agent, an appetite controlling agent, a uterus function controlling agent, a kidney function controlling agent, a prostate function controlling agent, a testis function controlling agent or a skeletal muscle function controlling agent;
(13) a method for screening an agonist or antagonist against the receptor protein containing the same or substantially the same amino acid sequence as that represented by the SEQ ID NO: 11, 12 or 13, which comprises using the peptide or a precursor, an amide, an ester or a salt thereof according to (1);
(14) a compound or a salt thereof obtained by the screening method according to (13);
(15) a memory function improving agent, an appetite controlling agent, a uterus function controlling agent, a kidney function controlling agent, a prostate function controlling agent, a testis function controlling agent or a skeletal muscle function controlling agent, which comprises the compound or a salt thereof according to (14);
(16) Use of (a) the peptide or a precursor, an amide, an ester or a salt thereof according to (1), (b) the DNA according to (3), or (c) the antibody according to (8) for producing a medicine having a memory function improving action, an appetite controlling action, a uterus function controlling action, a kidney function controlling action, a prostate function controlling action, a testis function controlling action or a skeletal muscle function controlling action; and
(17) a method for improving the memory function, or controlling the appetite, the uterus function, kidney function, prostate function, testis function or skeletal muscle function, which comprises administering to a mammal (a) the peptide or a precursor, an amide, an ester or a salt thereof according to (1), (b) the DNA according to (3), or the antibody according to (8).

### Brief Description of the Drawings

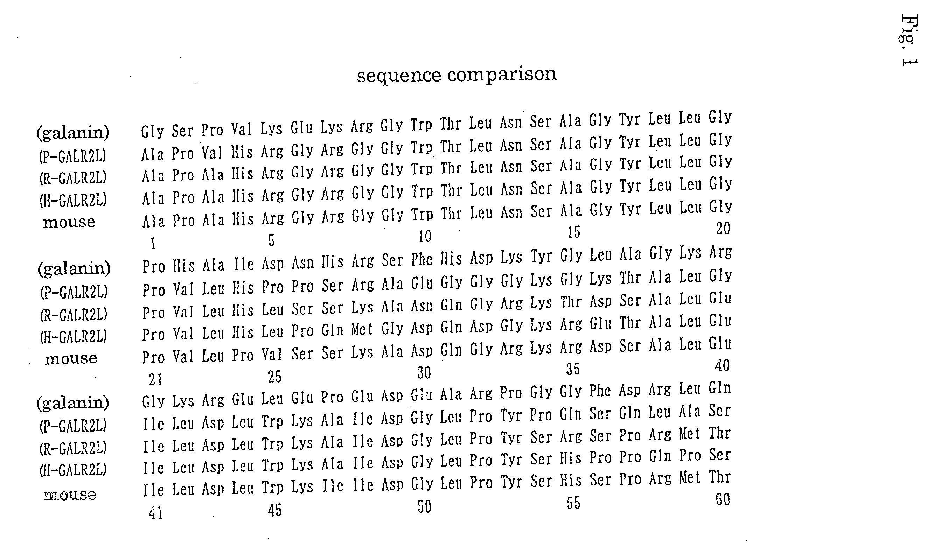

Fig. 1 shows the comparison of amino acid sequences of swine small intestine galanin (galanin), swine GalR2 ligand (P-GALR2L), mouse GalR2 ligand (R-GALR2L), human GalR2 ligand (H-GALR2L) and mouse GalR2 ligand according to the present invention.

### Detailed Description of the Invention

The phrase "substantially the same" used in the present specification means that the activity of a protein, for example an agonistic activity against a receptor, that is, an activity of a ligand for activating a receptor, and a binding activity of a ligand to a receptor, is substantially identical. Suppose that substitution, deletion, addition or insertion of an amino acid does not so often induce a large change in physiological and chemical properties of a peptide, then the peptide subjected to such substitution, deletion, addition or insertion would be called as substantially the same as a peptide that has not been subjected to these substitution, deletion, addition or insertion. A amino acid substituent that is substantially the same as the amino acid in the amino acid sequence in concern may be selected from, for example, other amino acids belonging to the class of the amino acid in concern. Examples of nonpolar (hydrophobic) amino acids include alanine, leucine, isoleucine, valine, proline, phenylalanine, tryptophane and methionine. Examples of polar (neutral) amino acids include glycine, serine, threonine, cysteine, tyrosine, asparagine and glutamine. Examples of positively charged (basic) amino acids include arginine, lysine and histidine. Examples of negatively charged (acidic) amino acids include aspartic acid and glutamic acid.

The peptide of the present invention has a binding ability to a galanin receptor. Preferably, the peptide has a galanin receptor activating action, and belongs to a ligand peptide other than galanin known in the art. The galanin receptor will be described hereinafter.

Examples of the peptide according to the present invention include, for example, those having the same or substantially the same amino acid sequence as that represented by the SEQ ID NO: 1 and a binding ability to a receptor protein (preferably an activating ability for the receptor protein) comprising the same or substantially the same amino acid sequence as that represented by the SEQ ID NO: 11, 12 or 13. The SEQ ID NO: 11, 12 and 13 denote the total amino acid sequence of rat GALR1 (galanin receptor type 1), rat GALR2 (galanin receptor type 2) and rat GALR3 (galanin receptor type 3), respectively.

The peptide of the present invention, and production methods and use thereof will be described hereinafter in more detail.

The above-mentioned peptide according to the present invention is derived from mouse and may be derived from tissues (for example, pituitary gland, pancreas, brain, kidney, liver, sexual grand, thyroid gland, gall bladder, bone marrow, adrenal gland, skin, muscle, lung, digestive tract, blood vessel, heart and testis) or cells of mouse, or may be a synthetic peptide.

The peptide according to the present invention includes a peptide containing amino acid sequences having a 90% or more (preferably 95% or more, more preferably 98% or more) homology with the amino acid sequence represented by the SEQ ID NO: 1.

The "substantially the same activity" means, for example, that the peptide is qualitatively identical in the activity for binding to the galanin receptors GALR1, GALR2 or GALR 3, the activity for activating the galanin receptors GALR1, GALR2 or GALR 3 or the ability for activating the signal transduction system such as arachidonic acid releasing, intracellular Ca²⁺ releasing, intracellular cAMP production inhibition, inositol phosphate producing (inhibiting), cell membrane potential changing, phosphorylation of intracellular protein and intracellular pH changing. Accordingly, quantitative factors such as intensities of these activities or molecular weight may be different among the peptides.

The following peptides are particularly exemplified as the peptide according to the present invention.

The peptides comprising substantially the same amino acid sequence include peptides having the same or substantially the same amino acid sequence as the sequence represented by the SEQ ID NO: 1 or the partial sequence thereof; and peptides having an amino acid sequence in which one or plural amino acids are substituted, deleted, added or inserted in the partial peptide. The examples include the peptides containing (1) an amino acid sequence in which one to ten amino acids, preferably one to five amino acids, and more preferably one to three amino acids are deleted from the amino acid sequence represented by the SEQ ID NO: 1 or the partial amino acid sequence thereof, (2) an amino acid sequence in which one to ten amino acids, preferably one to five amino acids, and more preferably one to three amino acids are added to (or inserted into) the amino acid sequence represented by SEQ ID NO: 1 or the partial amino acid sequence thereof, or (3) an amino acid sequence in which one to ten amino acids, preferably one to five amino acids, and more preferably one to three amino acids in the amino acid sequence represented by SEQ ID NO: 1 or the partial amino acid sequence thereof are substituted with other amino acids.

The "substantially the same amino acid sequence" includes an amino acid sequence having 90% or more, preferably 95% or more, and more preferably 98% or more of homology with the reference amino acid sequence.

In this specification, the left terminal of the peptide represents the N-terminal (amino terminal) and right terminal represents the C-terminal (carboxyl terminal) according to the customary representation of the peptide.

While the peptide of this invention usually has a carboxyl group (-COOH) or carboxylate (-COO⁻) at the C-terminal, it may be an amide (-COHN₂) or ester (-COOR). Examples of R in the ester include C₁₋₆ alkyl groups such as methyl, ethyl, n-propyl, isopropyl or n-butyl; C₃₋₈ cycloalkyl groups such as cyclopentyl or cyclohexyl; C₆₋₁₂ aryl groups such as phenyl or α-naphthyl; phenyl-C₁₋₂ alkyl such as benzyl, phenethyl or benzhydryl; or C₇₋₁₄ aralkyl group including α-naphthyl-C₁₋₂ alkyl such as α-naphthylmethyl; as well as pivaloyloxymethyl group frequently used as an oral administration esters.

When the peptides of the present invention have carboxylic groups or carboxylate groups other than the C-terminal, the peptides having amides or esters of these groups are also included in the peptide according to the present invention. The same ester groups as the C-terminal ester groups described above may be used as such ester groups.

As salts of the peptide of this invention, the salts with physiologically acceptable bases (for example alkali metals) or acids (organic and inorganic acids) may be used, among which physiologically acceptable acid addition salts are particularly preferred. Examples of these salts include salts with inorganic acids (e.g., hydrochloric acid, phosphate, hydrobromic acid and sulfuric acid), or salts with organic acids (e.g., acetic acid, formic acid, propionic acid, fumaric acid, maleic acid, succinic acid, tartaric acid, citric acid, malic acid, oxalic acid, benzoic acid, methanesulfonic acid and benzenesulfonic acid).

The peptide according to the present invention may be produced by the method purifying the peptide obtained from the tissue or cells of humans and other homeotherms, or may be synthesized in accordance with peptide synthesis method to be described hereinafter.

Alternatively, the peptide according to the present invention may be produced by cultivation of transformants comprising DNAs encoding the peptide. The DNAs encoding the peptide may be prepared according to the cloning methods known in the art [for example, the method described in Molecular Cloning (2nd ed.; J. Sambrook et al., Cold Spring Harbor Lab. Press, 1989)].

The cloning methods include (1) a method obtaining a transformant which comprises a DNA encoding the peptide by a hybridization method from a cDNA library using a DNA probe or DNA primer designed on the basis of the amino acid sequence of the peptide, or (2) a method obtaining a transformant which comprises a DNA encoding the peptide by a PCR method using a DNA primer designed on the basis of the amino acid sequence of the peptide.

When the peptide according to the present invention is produced from tissues or cells of humans and other homeotherms, the peptide may be extracted from the homogenized tissues or cells of humans and other homeotherms with an acid or alcohol, and then purified and isolated by salting out and dialysis in combination with chromatography such as gel filtration chromatography, reversed phase chromatography, ion-exchange chromatography and affinity chromatography.

The peptide according to the present invention as described above may be produced (1) according to the peptide synthesis method known in the art, or (2) by fragmentation of a peptide containing the peptide of the present invention with an appropriate peptidase.

The peptide may be synthesized by either a solid phase method or a liquid phase method. The desired peptide may be synthesized by condensing a partial peptide or amino acids that can constitute the peptide of the present invention with remaining moiety of the peptide, and eliminating protective groups of the product, if any. The known condensation methods and elimination methods of the protective groups, for example, include the following methods described in (1) and (2):
(1) M. Bodanszky and M. A. Ondetti, Peptide Synthesis, Interscience Publishers, New York, 1966; and
(2) Schroeder and Luebke, The Peptide, Academic Press, New York, 1965

After the reaction, the peptide of the present invention can be isolated and purified by conventional purification methods, for example, combining solvent extraction, distillation, column chromatography, liquid chromatography and recrystallization. When the peptide obtained by the methods above is a free peptide, it may be converted into an appropriate salts by a method known in the art, and when the peptide is obtained as a salt, on the contrary, it may be converted into a free peptide by a method known in the art.

A commercially available peptide synthesis resin suitable for forming an amide may be used for the amide derivative of the peptide of the present invention. Examples of these resins include a chloromethyl resin, hydroxymethyl resin, benzhydrylamine resin, aminomethyl resin, 4-benzyloxy benzylalcohol resin, 4-methylbenzhydrylamine resin, PAM resin, 4-hydroxymethyl methylphenylacetamidemethyl resin, polyacrylamide resin, 4-(2',4'-dimethoxyphenyl-hydroxymethyl)phenoxy resin, and 4-(2',4'-dimethoxyphenyl-Fmoc-aminoethyl)phenoxy resin. Amino acids of which α-amino group and side chain functional groups are properly protected, are condensed on a resin in order of the amino acid sequence of the desired peptide according to various condensation methods known in the art, per se, using these resins described above. The various protective groups are eliminated concomitantly with excision of the peptide from the resin at the final stage of the reaction, followed by forming intramolecular disulfide bonds, if necessary, in a highly diluted solution to obtain the desired peptide.

While various kinds of activation reagents applicable for the peptide synthesis may be used for the condensation of protected amino acids above, carbodiimides are particularly suitable for the purpose. Examples of the carbodiimides include DCC, N,N'-diisopropyl carbodiimide and N-ethyl-N'-(3-dimethylaminopropyl)carbodiimide. For activation using these reagents, a protected amino acid may be directly added to the resin together with a racemization preventive additive (e.g., HOBt, HOOBt etc), or may be added to the resin after the amino acid that has been previously protected as a symmetrical acid anhydride, a HOBt eater or a HOOBt ester is activated. Solvents that can be used for the activation of the protected amino acid or for the condensation with the resin may be properly selected from solvents that have been known to be available for the peptide condensation reaction. They include, for example, acid amides such as N,N-dimethylformamide, N,N-dimetylacetamide and N-methylpyrrolidone; halogenated hydrocarbons such as methylene chloride and chloroform; alcohols such as trifluoroethanol; sulfoxides such as dimethylsulfoxide; tertiary amines such as pyridine; ethers such as dioxane and tetrahydrofuran; nitriles such as acetonitrile and propyonitrile; esters such as methyl acetate and ethyl acetate; and an appropriate mixture thereof. The reaction temperature is appropriately selected from a temperature range available for forming peptide bonds, and is usually selected in a range of about - 20°C to about 50°C. The activated amino acid derivative is usually used in an amount of about 1.5 to abut 4 times excess of equivalence. The condensation reaction may be sufficiently advanced by repeating the condensation reaction without removing the protective groups, when the condensation reaction is revealed to be insufficient by a test using a ninhydrin reaction. When a sufficient condensation is not obtained even after repeated reactions, unreacted amino acids may be acetylated using acetic anhydride or acetyl imidazole so that the unreacted amino acids may not affect the succeeding reactions.

The protective groups of the amino group of the amino acid as a starting material include Z, Boc, tert-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulfenyl, diphenylphosphinothioyl and Fmoc. Examples of the protective groups of the carboxylic group include, for example, R as described above such as C₁₋₆ alkyl group, C₃₋₈ cycloalkyl group and C₇₋₁₄ aralkyl group, as well as 2-adamantyl, 4-nitrobenzyl, 4-methoxybenzyl, 4-chlorobenzyl, phenacyl groups, benzyloxycarbonylhydrazide, tert-butoxycarbonylhydrazide and tritylhydrazide.

The hydroxyl groups of serine and threonine may be protected, for example, by esterification or etherification. Groups suitable for esterification include, for example, lower (C₁₋₆) alkanoyl groups such as acetyl group; aroyl groups such as benzoyl group; and groups derived from carbonic acid such as benzyloxycarbonyl group and ethoxycarbonyl group. Groups suitable for etherification include, for example, benzyl group, tetrahydropiranyl group and tert-butyl group.

Protective groups for the phenolic hydroxyl group of tyrosine include, for example, Bzl, Cl₂-Bzl, 2-nitrobenzyl, Br-Z and tert-butyl.

Protective groups for imidazole of histidine include Tos, 4-methoxy-2,3,6-trimethylbenzenesulfonyl, DNP, benzyloxymethyl, Bum, Boc, Trt and Fmoc.

Examples of activated carboxyl groups in the starting material include, for example, corresponding acid anhydrides, azides, and activated esters [esters of alcohols (for example, pentachlorophenol, 2,4,5-trichlorophenol, 2,4-dinitrophenol, cyanomethylalcohol, p-nitrophenol, HONB, N-hydroxysuccimide, N-hydroxyphthalimide and HOBt)]. Examples of the activated amino groups in the starting material include the corresponding phosphoric acid amides.

Methods for removing (eliminating) the protective groups include, for example, catalytic reduction under a hydrogen stream in the presence of a catalyst such as Pd black or Pd carbon; acid treatment with anhydrous hydrogen fluoride, methanesulfonic acid, trifluoromethanesulfonic acid or trifluoroacetic acid, or a mixture thereof; base treatment with diisopropyl ethylamine, triethylamine, piperidine or piperazine; and reduction with sodium in liquid ammonia. While the elimination reaction by the acid treatment above is usually carried out at -20°C to 40°C, it is effective in the acid treatment to add a cation trapping agent such as anisole, phenol, thioanisole, m-cresol, p-cresol, dimethylsulfide, 1,4-butanedithiol and 1,2-ethanedithiol. A 2,4-dinitrophenyl group that is used as an imidazole protective group of histidine is removed by a thiophenol treatment, and a formyl group that is used as an indole protective group of tryptophane may be removed by an alkaline treatment with a dilute sodium hydroxide solution or dilute ammonia solution, in addition to deprotection by an acid treatment in the presence of 1,2-ethanedithiol and 1,4-butanedithiol described above.

Any groups and methods known in the art may be appropriately selected for protection and protective groups of functional groups that should not involve in the reaction with the starting materials, elimination of the protective group, and activation of the functional groups involved in the reaction.

Another method for obtaining an amide of the peptide according to the present invention include: forming an amide of an α-carboxyl group of an amino acid at the carboxyl terminal; elongating a peptide chain toward the amino terminal with a desired chain length; preparing a peptide in which only the protective group of the α-amino group at the N-terminal is removed, and a peptide (or amino acid) in which only the protective group of the carboxyl group at the C-terminal is removed; and allowing these two peptides to condense in a mixed solvent described above. Details of the condensation reaction are as same as described above. After purification of the protected peptide obtained by condensation, all the protective groups are removed by the above-mentioned methods to obtain a desired crude peptide. The crude peptide may be purified by taking advantage of various purification methods known in the art, and an amide of the desired peptide can be obtained by freeze-drying main fractions.

For obtaining an ester of the peptide according to the present invention, an amino acid ester is formed by condensation of the α-carboxylic group of an amino acid at the carboxylic terminal with a desired alcohol, followed by the same process as used in the amide of the peptide to obtain the desired ester of the peptide.

Any peptides may be included in the peptide according to the present invention so long as the peptide has similar actions (for example, GALR1 agonistic activity, GALR2 agonistic activity, GALR3 agonistic activity and the like) to those of the peptides having the same or substantially the same amino acid sequence as the amino acid sequence represented by SEQ ID NO: 1 or the partial sequence thereof. Examples of such peptides include, for example, a peptide with the amino acid sequence in which one to five amino acids in the amino acid sequence represented by SEQ ID NO:1 or a partial sequence thereof are deleted or substituted or one to five amino acids are added to (inserted into) the amino acid sequence represented by SEQ ID NO:1 or a partial sequence thereof.

The peptide precursor according to the present invention may include any peptides so long as the ligand peptide of the present invention is encoded as a partial sequence of the peptide.

Examples of the peptide precursor according to the present invention include, for example, a peptide (or a polypeptide) comprising the same or substantially the same amino acid sequence as that represented by SEQ ID NO: 2.

Herein, the "substantially the same amino acid sequence" includes an amino acid sequence having 80% or more, preferably 90% or more, more preferably 95% or more, and most preferably 98% or more of homology with the amino acid sequence represented by SEQ ID NO: 2.

The "substantially the same amino acid sequence" includes (i) an amino acid sequence in which one to 20 amino acids, preferably one to 10 amino acids, and more preferably one to five amino acids in the amino acid sequence represented by SEQ ID NO: 2 are substituted with other amino acids, (ii) an amino acid sequence in which one to 20 amino acids, preferably one to 10 amino acids, and more preferably one to five amino acids in the amino acid sequence represented by SEQ ID NO; 2 are deleted, and (iii) an amino acid sequence in which one to 20 amino acids, preferably one to 10 amino acids, and more preferably one to five amino acids are added to (inserted into) the amino acid sequence represented by SEQ ID NO: 2. The site for "substitution, deletion or addition (or insertion)" is not particularly restricted so long as the site is out of the region where the ligand peptide of the present invention is encoded as a partial sequence thereof.

Examples of the peptides (or polypeptides) comprising the same or substantially the same amino acid sequence as that represented by SEQ ID NO: 2 include, for example, peptides (or polypeptides) comprising the amino acid sequence represented by SEQ ID NO: 1 as well as (i) an amino acid sequence in which one to 20 amino acids, preferably one to 10 amino acids, and more preferably one to five amino acids in the amino acid sequence represented by SEQ ID NO: 2 are substituted with other amino acids, (ii) an amino acid sequence in which one to 20 amino acids, preferably one to 10 amino acids, and more preferably one to five amino acids in the amino acid sequence represented by SEQ ID NO: 2 are deleted, and (iii) an amino acid sequence in which one to 20 amino acids, preferably one to 10 amino acids, and more preferably one to 5 amino acids are added to (inserted into) the amino acid sequence represented by SEQ ID NO: 2.

The peptide of the present invention can be used as an antigen for preparing an anti-ligand peptide antibody. The peptide of the present invention above as well as a partial peptide such as a N-terminal peptide, C-terminal peptide and central portion peptide of the peptide of the present invention may be used as such antigens.

While a peptide independently containing each domain of the peptide of the present invention may be used as a partial peptide, the peptide may be a partial peptide concomitantly containing plural domains.

Since the partial peptide is only required to be enough for using as an antigen for preparing the anti-ligand peptide antibody, the partial peptide above may also contain (i) a partial peptide in which 1 to several (preferably 1 to 5, more preferably 1 or 2) other amino acid residues are added to (or inserted into) the partial peptide such as a N-terminal peptide, C-terminal peptide and central portion peptide of the above-mentioned peptide of the present invention, (ii) a partial peptide in which 1 to several (preferably 1 to 5, more preferably 1 or 2) amino acid residues of the partial peptide such as a N-terminal peptide, C-terminal peptide and central portion peptide of the above-mentioned peptide of the present invention are deleted, (iii) a partial peptide in which 1 to several (preferably 1 to 5, more preferably 1 or 2) constitutive amino acids of the partial peptide such as a N-terminal peptide, C-terminal peptide and central portion peptide of the above-mentioned peptide of the present invention are substituted with other amino acid residues.

In particular, the partial peptide described above includes, for example, a peptide containing a partial amino acid sequence comprising NO. 24 amino acid (Pro) through No. 30 amino acid (Asp), a partial amino acid sequence comprising No. 32 amino acid (Gly) through No. 37 amino acid (Ser) and a partial amino acid sequence comprising No. 55 amino acid (His) through NO. 60 amino acid (Thr) from the N-terminal of the amino acid sequence represented by SEQ ID NO: 1.

The C-terminal of the partial peptide in the present specification may be an amide (-CONH₂) or an ester (-COOR). The ester groups are the same as those in the above-mentioned peptides. When the partial peptide has the carboxylic groups or carboxylates at the sites other than the C-terminal, those in which these carboxylic groups or carboxylates are amidated or esterified are also included in the partial peptide of the present invention. Such esters include, for example, the esters of C-terminal described above.

The partial peptide of the peptide according to the present invention may have the activities, per se, involved in the peptide of the present invention (for example an activation action against the galanin receptor). The peptide of the present invention or the partial peptide thereof may be a fused protein with a protein whose functions or properties are well known.

The same salts as the above-mentioned peptide salts may be used as the salts of the partial peptide of the peptide according to the present invention.

The partial peptide of the peptide of the present invention, or an amide, an ester or a salt thereof, may be produced by the same synthetic method as used in the above-mentioned peptide, or by severing the peptide of the present invention with an appropriate peptidase.

Any DNAs may be used as the DNA encoding the peptide of the present invention, so long as the DNA comprises nucleotide sequences encoding such peptides having a binding ability to a receptor protein (preferably, having an ability for activating the receptor protein) which contains the same or substantially the same amino acid sequence as that represented by SEQ ID NO: 1, preferably contains the same or substantially the same amino acid sequence as that represented by SEQ ID NO: 11, 12 or 13.

In addition, any DNAs encoding the peptide of the present invention exemplified above may be used.

The DNAs may be either genome DNAs, genome DNA libraries, cDNAs derived from the foregoing tissues and cells, cDNA libraries derived from the foregoing tissues and cells or synthetic DNAs. The vector to be used in the library may be any one of bacteriophage, plasmid, cosmid or phagemid. The DNAs may be amplified directly by a Reverse Transcriptase Polymerase Chain Reaction (referred to as a RT-PCR method hereinafter) using RNA fractions prepared from the foregoing tissue and cells.

Specifically, available are the DNAs such as (1) a DNA comprising the DNA encoding the peptide containing the same or substantially the same amino acid sequence as that represented by SEQ ID NO: 1, (2) a DNA derived from a mammal which hybridizes with a sequence defined by (1) under a stringent condition, and (3) a DNA encoding a polypeptide having the same amino acid sequence as those in (1) and (2), although it does not hybridize with the DNAs defined in (1) and (2) due to degeneracy of genetic codes. The DNAs may be hybridized by a method known in the art, per se, or a similar method thereof. The stringent condition above comprises, for example, a temperature of 42°C, 50% formamide, 4 × SSPE (1 × SSPE = 150 mM NaCl, 10 mM NaH₂PO₄•H₂O, 1 mM EDTA pH7.4), 5 × Denhardt solution, and 0.1% SDS.

Specific examples of the DNA, which comprises the DNA encoding the peptide containing the same or substantially the same amino acid sequence as that represented by SEQ ID NO: 1, include the DNA comprising the nucleotide sequence represented by SEQ ID NO: 3.

Further, among the DNAs encoding the peptides of the present invention or a partial peptide thereof, a DNA fragment which comprises a partial nucleotide sequence having 1 to 40 (preferably 1 to 30, more preferably one to 20) nucleotides, may be preferably used as a DNA detection probe.

Any DNAs may be used as the DNA encoding the precursor of the peptide of the present invention, so long as the DNAs comprise DNAs encoding the precursor of the peptide of the present invention, specifically, the peptide (polypeptide) containing the same or substantially the same amino acid sequence as that represented by SEQ ID NO: 2.

A specific example of the DNA containing a DNA, which encode the peptide (polypeptide) containing the same or substantially the same amino acid sequence as that represented by SEQ ID NO: 2, includes a DNA containing the DNA represented by SEQ ID NO: 20.

The DNA encoding the peptide of the present invention can also be produced by genetic engineering method described below. The genetic engineering method includes, for example, the method described in Molecular Cloning (2nd ed.; J. Sambrook et al., Cold Spring Harbor Lab. Press, 1989). When a commercially available library or kit is used, the method indicated in the attached instruction may be referenced.

The DNA encoding the peptide of the present invention may be fractionated by hybridizing a probe which is a labeled DNA fragment synthesized based on the amino acid sequence or a part thereof, with a library of a genome DNA or cDNA.

For cloning the DNA perfectly encoding the peptide of the present invention, the DNA may be fractionated by amplifying the desired DNA from a genome DNA or cDNA library by a PCR method known in the art, per se, utilizing a synthetic DNA primer having the partial nucleotide sequence of the peptide of the present invention, or by hybridizing a DNA integrated in an appropriate vector, with a labeled DNA fragment or synthetic DNA having a partial or entire code region of the peptide of the present invention.

The DNA encoding the cloned peptide of the present invention may be used, depending on the object, directly, by digesting with a restriction enzyme, if necessary, or by adding a linker. The DNA may have an ATG sequence as a translation initiating codon at the 5'-terminal thereof, or a TAA, TGA or TAG sequence as a translation terminating codon at the 3'-terminal thereof. These translation initiating codon and translation terminating codon may be added by using an appropriate synthetic DNA adapter.

The expression vector for the peptide of the present invention can be produced, for example, by (a) severing a desired DNA fragment from the DNA encoding the peptide of the present invention, and (b) coupling the DNA fragment to a downstream of a promoter in an appropriate expression vector.

The vectors available include plasmids derived from Escherichia coli (e.g., pBR322, pBR325, pUC12, pUC13), plasmids derived from Bacillus subtilis (e.g., pUB110, pTP5, pC194), plasmids derived from yeast (e.g., pSH19, pSH15), bacteriophage such as λ-phage and animal viruses such as retrovirus, vacciniavirus and baculovirus.

The promoters used in the present invention may be any one that properly corresponds to the hosts used for expression of genes.

When the host for transformation is animal cells, promoters available include a promoter derived from SV40, a retrovirus promoter, a metallothionein promoter, a heat-shock promoter, a cytomegalovirus promoter and SRα-promoter. When the host belongs to genus Escherichia, preferable promoters include a trp promoter, T7 promoter, lac promoter, recA promoter, λPL promoter and lpp promoter. When the host belong to genus Bacillus, preferable promoters include a SPO1 promoter, SPO2 promoter and penP promoter. When the host is yeast, preferable promoters include a PHO5 promoter, PGK promoter, GAP promoter, ADH1 promoter and GAL promoter. When the host is insect cells, preferable promoters include a polyhedrin promoter and P10 promoter.

Other expression vectors available other than those described above may contain an enhancer, a spricing signal, a poly A addition signal, a selection marker and a SV40 replication origin (sometimes abbreviated as SV40ori hereinafter). Examples of the selection markers include, for example, a dihydrofolic acid reductase (sometimes abbreviated as dhfr hereinafter) gene [methotrexate (MTX) resistant], an ampicillin resistant gene (sometimes abbreviated as Amp^{r} hereinafter), and a neomycin resistant gene (sometimes abbreviated as Neo^{r} hereinafter, G418 resistant). When a DHFR gene is used as a selection marker using CHO (dhfr⁻) cells, the desired gene may be selected by using a medium containing no thymidine.

A signal sequence fitted to the host is added to the N-terminal of the peptide or a partial peptide thereof, if necessary. The sequence available include a phoA signal sequence and an OmpA signal sequence when the host belongs to genus Escherichia; an α-amylase signal sequence and a subtilisin signal sequence when the host belongs to genus Bacillus; a mating factor α (Mfα) signal sequence and an invertase signal sequence when the host is yeast; and an insulin signal sequence, an α-interferon signal sequence and an antibody molecule signal sequence when the host is an animal cell.

A transformant may be created using a vector comprising the DNA encoding the peptide constructed as described above.

For example, genus Escherichia, genus Bacillus, yeast, insects or insect cells, and animal cells may be used for the host.

Genus Escherichia available includes Escherichia coli K12•DH1 (Proc. Natl. Acad. Sci. USA, 60, 160 (1968)), JM103 (Nucleic Acids Research, 9, 309 (1981)), JA221 (Journal of Molecular Biology, 120, 517 (1978)), HB101 (Journal of Molecular Biology, 41, 459 (1969)), and C600 (Genetics, 39, 440 (1954)).

Genus Bacillus available includes, for example, Bacillus subtilis MI114 (Gene, 24, 255 (1983)) and 207-21 (Journal of Biochemistry, 95, 87 (1984)).

Yeast available includes, for example, Saccharomyces cerevisiae AH22, AH22R⁻, NA87-11A, DKD-5D and 20B-12.

Insects available include, for example, larvae of Bombyx mori N (Maeda et al., Nature, 315, 592 (1985)).

When the virus is AcNPV, insect cells available include, for example, established cell lines derived from larvae of Spodoptera frugiperda (Sf cells), MG1 cells derived from midgut of Trichoplusia ni, High Five™ cells derived from eggs of Trichoplusia ni, cells derived from Mamestra brassicae or cells derived from Estigmena acrea. Established cell lines derived from Bombyx mori N (BmN cells) are used when the virus is BmNVP. As the said Sf cells, for example, Sf9 cells (ATCC CRL1711) and Sf21 cells (Vaughn, J. L. et al, in Vitro, 13, 213-217 (1977)).

Animal cells available include, for example, monkey COS-7 cells and Vero cells, Chinese hamster CHO cells, DHFR gene deficient Chinese hamster cells CHO (CHO/dhfr⁻ cells), mouse L-cells, mouse 3T3 cells, mouse myeloma cells, human HEK 293 cells, human FL-cells, 293 cells, C127 cells, BALB3T3 cells and Sp-2/O cells.

Cells belonging to genus Escherichia are transformed according to the methods, for example, described in Proc. Natl. Acad. Sci. USA, 69, 2110 (1972), and Gene, 17, 107 (1982).

Cells belonging to genus Bacillus are transformed according to the method, for example, described in Molecular and General Genetics, 168, 111 (1979).

Yeast cells are transformed according to the method, for example, described in Proc. Natl. Acad. Sci. USA, 75, 1929 (1978).

Insect cells and insects are transformed according to the method, for example, described in Bio/Technology, 6, 47-55 (1988).

Animal cells are transformed according to the method, for example, described in Virology, 52, 456 (1973).

Examples of the method for introducing an expression vector into cells include, for example, a lipofection method (Felgner, P. L. et al., Proceedings of the National Academy of Sciences of the United States of America, 84, 7413 (1987)), a calcium phosphate method (Graham, F. L. and van der Eb, A.J., Virology, 52, 456-467 (1973)), and an electroporation method (Neumann, E. et al., EMBO J., 1, 841-845 (1982)).

Thus, a transformant transformed with an expression vector comprising the DNA encoding the peptide of the present invention can be obtained.

The method for stable expression of the peptide of the present invention using animal cells also include a method for selecting the cells, in which the expression vector introduced into the animal cells are chromosomally integrated, by clone selection. Practically, transformants are selected using the selection marker above as an index. A stable animal cell strain highly expressing the peptide of the present invention may be obtained by repeating clone selection for the animal cells obtained thus by using the selection marker. When the dhfr gene is used as a selection marker, the cells are cultivated by slowly increasing the concentration of MTX to select MTX resistant cells, and the dhfr gene as well as the DNA encoding the peptide of the present invention are amplified in the cell, thereby enabling a high expression animal cell strain to be obtained.

The peptide according to the present invention can be produced by cultivating the transformant above under a condition capable of expressing the DNA encoding the peptide of the present invention, followed by producing and accumulating the peptide of the present invention.

When the transformant of which host belongs to genus Escherichia or genus Bacillus is incubated, a liquid medium is suitable for use in cultivation, and carbon and nitrogen sources, inorganic substances and the like necessary for the growth of the transformant may be incorporated in the medium. Examples of the carbon source include, for example, glucose, dextrin, soluble starch and sucrose, and examples of the nitrogen source include, for example, inorganic or organic substances such as ammonium slats, nitrate salts, corn steep liquor, peptone, casein, meat extract, soybean flake and potato extract. And calcium chloride, sodium dihydrogen phosphate, magnesium chloride and the like may be exemplified as the inorganic substances. Other nutrients such as yeast extract, vitamins and growth promoters may be also added. The preferable pH of the medium is about 5 to 8.

The preferable culture medium for cultivating the genus Escherichia is, for example, a M9 medium containing glucose and casamino acid (Journal of Experiments in Molecular Genetics, pp431-433, Cold Spring Harbor Laboratory, New York, 1972). An agent such as 3β-indolyl acrylic acid may be added for allowing the promoter to function efficiently, if necessary.

When the host belongs to genus Escherichia, the cells are usually cultivated at about 15 to 43°C for about 3 to 24 hours, if necessary, with aeration and stirring.

When the host belongs to genus Bacillus, the cells are usually cultivated at about 30 to 40°C for about 6 to 24 hours, if necessary, with aeration and stirring.

Examples of the culture medium of a transformant derived from yeast as a host include, for example, a Burkholder's minimal medium (Bostian, K. L. et al., Proc. Natl. Acad. Sci. USA, 77, 4505 (1980)), and a SD medium containing 0.5% casamino acid (Bitter, G. A et al., Proc. Natl. Acad. Sci. USA, 81, 5330 (1984)). The medium is preferably adjusted to pH 5 to 8. The cells are usually cultivated at about 20 to 35°C for about 24 to 72 hours, if necessary, with aeration and stirring.

Examples of the culture medium of a transformant derived from insect cells as hosts include Grace's Insect Medium (Grace, T. C. C., Nature, 195, 788 (1962)) optionally supplemented with 10% bovine serum and the like. The medium is preferably adjusted to pH 6.2 to 6.4. The cells are usually cultivated at about 27°C for about 3 to 5 days, if necessary, with aeration and stirring.

Examples of the culture medium of a transformant derived from animal cells as hosts include, for example, a MEM medium (Science, 122, 501 (1952)), a DMEM medium (Virology, 8, 396 (1959)), RPMI 1640 medium (The Journal of the American Medical Association, 199, 519 (1967)), and 199 medium (Proceeding Of the Society for the Biological Medicine, 73, 1 (1950)), which contain about 5 to 20% fetal bovine serum. The medium is preferably adjusted to about pH 6 to 8. The cells are usually cultivated at about 30 to 40°C for about 15 to 60 hours, if necessary, with aeration and stirring.

When CHO (dhfr⁻) cells and dhfr gene are used as selection markers, in particular, it is preferred to use a DMEM medium containing dialyzed fetal bovine serum with substantially no thymidine.

The following methods may be employed for separation and purification of the peptide of the present invention from the cultures described above.

In extracting the peptide of the present invention from the cultured bacteria or cells, the cells are collected by a method known in the art, and suspended in an appropriate buffer solution. After destruction of the bacteria or cell using ultrasonic wave, lysozyme and/or by freezing and melting, a crude extract of the peptide is obtained by centrifugation or filtration. Protein denaturants such as urea and guanidine hydrochloride, and surfactants such as Triton X-100 (registered trade mark, abbreviated as TM hereinafter) may be contained in the buffer solution.

When the peptide is secreted in the culture medium, the supernatant is separated from the bacteria or cells by a method known in the art, and the supernatant is collected.

The culture supernatant thus obtainedor the peptide of the present invention contained in the extract may be purified by an appropriate combination of separation and purification methods known in the art. These methods known in the art include a method taking advantage of solubility such as salting-out and solvent precipitation, a method mainly taking advantage of molecular weight differences such as dialysis, ultrafiltration, gel filtration and SDS-polyacrylamide gel electrophoresis, a method taking advantage of charge differences such as ion-exchange chromatography, a method taking advantage of differences in specific affinity such as affinity chromatography, a method making use of differences in hydrophobicity such as reversed phase high performance liquid chromatography and a method making use of differences in isoelectric point such as an isoelectric electrophoresis method and focusing chromatography.

When the peptide of the present invention is obtained as a free peptide, it may be converted into a salt thereof by a method known in the art or a method similar to it. When the peptide is obtained as a salt, on the other hand, it may be converted into a free peptide or a different salt thereof by a method known in the art or a method similar to it.

The peptide according to the present invention produced by the transformant may be optionally modified or a part of the peptide may be eliminated before or after purification by using an appropriate protein modifying enzyme. Examples of the protein modifying enzyme available include, for example, trypsin, chymotrypsin, arginine endopeptidase, protein kinase and glycosidase.

The peptide of the present invention formed may be detected by an enzyme immunoassay using an antibody specific to the peptide.

The DNA encoding the peptide of the present invention or the peptide of the present invention can be applied for (1) synthesis of a part of, or a whole length of a ligand for the galanin receptor protein, (2) search of physiological actions involved in the peptide of the present invention, (3) preparation of a synthetic oligonucleotide probe or PCR primer, (4) acquisition of ligands for G-protein conjugate receptor proteins and DNAs encoding precursor proteins, (5) development of receptor binding assay system using expression systems of recombinant receptor proteins and screening of pharmaceutical candidate compound, (6) acquisition of antibodies and antiserum, (7) development of diagnostics using DNA, RNA, antibodies or antiserum, (8) development of medicines such as a memory function improving agent (intelligence-tropic drug), an appetite controlling agent, a diabetes curative medicine, pituitary gland function improving agent, a uterus function controlling agent, a kidney function controlling agent, a prostate function controlling agent, a testis function controlling agent, or a skeletal muscle function controlling agent (preferably a memory function improving agent (intelligence-tropic drug), an appetite controlling agent, a uterus function controlling agent, a kidney function controlling agent, a prostate function controlling agent, a testis function controlling agent or a skeletal muscle function controlling agent), and (9) gene therapy.

Particularly, the G-protein conjugate receptor agonist or antagonist specific to homeotherms such as human may be screened using a receptor binding assay system which make use of expression systems of the recombinant G-protein conjugate receptor proteins described hereinafter, and the agonist and antagonist can be utilized as preventive and curative medicines of various diseases.

With respect to (8) above, since the peptide of the present invention or the DNA encoding the peptide is recognized as a ligand by the galanin receptor (GALR) protein expressed in hippocampus, hypothalamus, uterus, kidney, prostate, skeletal muscle, pancreas, testis, spleen, heart and pituitary gland, it is useful as safe and low toxicity medicines. Accordingly, the peptide may be used, for example, as a memory function improving agent (intelligence-tropic drug), an appetite controlling agent, a diabetes curative medicine, pituitary gland function improving agent, a uterus function controlling agent, a kidney function controlling agent, a prostate function controlling agent, or a skeletal muscle function controlling agent (preferably a memory function improving agent (intelligence-tropic drug), an appetite controlling agent, a uterus function controlling agent, a kidney function controlling agent, a prostate function controlling agent, or a skeletal muscle function controlling agent).

Since the polypeptide or the DNA encoding the polypeptide of the present invention has a specific LH blood level increasing action (LH secretion promoting action), while galanin highly homologous with the polypeptide of the present invention shows no LH secretion promoting action, the LH secretion promoting action is considered to be an action specific to the polypeptide of the present invention which is not found in galanin.

Having LH secretion promoting action, the peptide or the DNA encoding the peptide of the present invention may be used as preventive and curative medicines for various diseases involved in LH secretion insufficiency (for example, infertility, paramenia, dysmenorrhea, amenorrhea, premenstrual syndrome, climacteric disorder, hypopituitarism or obesity). In other words, the LH secretion controlling agent containing the polypeptide or the DNA encoding the polypeptide of the present invention may be used as a LH secretion promoting agent for prevention and treatment of infertility, paramenia, dysmenorrhea, amenorrhea, premenstrual syndrome, climacteric disorder, hypopituitarism or obesity. The LH secretion controlling agent containing the polypeptide or the DNA encoding the polypeptide of the present invention is also considered to be particularly effective for prevention and therapy of various diseases originating from abnormality in the leptin receptor (for example, obesity and infertility).

The polypeptide of the present invention also has an action for suppressing LH secretion as a result of desensitization against LH secretion when the amount of administration of the polypeptide or the DNA encoding the polypeptide of the present invention is increased, since the polypeptide of the present invention has a high affinity with its receptor protein. Consequently, the LH secretion controlling agent containing the polypeptide or the DNA encoding the polypeptide of the present invention may be also used as a LH secretion suppressing agent for prevention and treatment of various diseases involved in excess secretion of LH (for example, prostate cancer, prostatic hypertrophy, early onset of puberty and tumor of LH production pituitary gland).

The peptide of the present invention or the DNA encoding the peptide may be administered as a medicine by common methods. For example, it may be administered orally as a tablet which is, if necessary, sugar coated or enteric coated, capsule, elixir and microcapsule, or parenterally as an injection solution such as an aseptic solution or suspension in a pharmaceutically acceptable solution such as water and other solvents. For example, a medicine may be produced by mixing the compound or a salt thereof with a pharmaceutically acceptable carrier, flavoring agent, excipient, vehicle, preservative, stabilizer and binder in a unit dosage required for generally accepted manufacturing practice. The effective amount of ingredients in these formulations is controlled to be available for prescribed proper dosage.

When the DNA of the present invention is used, the DNA may be administered alone, or by a conventional method after inserting it into an appropriate vector such as a retrovirus vector, adenovirus vector and adenovirus associated virus vector.

Additives capable of mixing into a tablet or capsule include, for example, binders such as gelatin, corn starch, tragacanth and gum arabic, excipient such as crystalline cellulose, swelling agents such as corn starch, gelatin and alginic acid, lubricants such as magnesium stearate, sweeteners such as sucrose, lactose and saccharin, and flavoring agents such as peppermint, akamono oil and cherry. When the formulation unit is a capsule, a liquid carrier such as an oil may be incorporated in addition to the materials described above. An aseptic composition for injection may be formulated by dissolving or dispersing an active ingredient in a vehicle such as injectable water and natural oils such as sesame oil and coconut oil according to the usual preparation method.

An aqueous injectable solution includes, for example, a physiological saline solution and an isotonic solution containing glucose and other auxiliary agents (e.g., D-sorbitol, D-mannitol and sodium chloride), and appropriate solubilizing agents such as alcohols (e.g., ethanol), polyalcohols (e.g., propylene glycol and polyethylene glycol) and non-ionic surfactants (e.g., polysorbate 80(TM), HCO-50) may be used together. Oily solutions include sesame oil and soybean oil, and benzyl benzoate and benzyl alcohol may be used together as solubiling agents.

Also compounded are a buffer solution (e.g., phosphate buffer or sodium acetate buffer), an indolent agent (e.g., benzalkonium chloride or procaine hydrochloride), a stabilizer (e.g., human serum albumin or polyethylene glycol), a preservative (e.g., benzyl alcohol or phenol) and an antioxidant. The injection solution formulated is usually filled in an appropriate ampoule.

Since the formulation thus obtained is safe and low-toxic, it may be administered to human and mammals (for example mouse, rat, guinea pig, rabbit, sheep, swine, bovine, cat, dog and monkey).

While the dosage of the peptide of the present invention or the DNA encoding the peptide may be different depending on the disease conditions, it is about 0.1 to 100 mg, preferably about 1.0 to 50 mg, and more preferably about 1.0 to 20 mg a day for an adult (with a body weight of 60 kg) for oral administration of the peptide. In the case of parenteral administration (e.g., administration by intravenous injection), the dosage as an injection formulation is about 0.01 to 30 mg, preferably about 0.1 to 20 mg, and more preferably about 0.1 to 10 mg a day for an adult (with a body weight of 60 kg), although it depends on the administration subject, organs to be administered, disease conditions and administration methods.

The dosage to other mammals may be determined by converting the dosage to human with a body weight of 60 kg into the dosage to a mammal considering the body weight of the mammal to be administered.

In the present invention, the galanin receptor may be any protein which is a G-protein conjugate receptor protein derived from all the tissues (e.g., pituitary gland, pancreas, brain, kidney, liver, sexual gland, thyroid gland, gallbladder, bone marrow, adrenal gland, skin, muscle, lung, digestive tract, blood vessel, heart, etc.) or cells of human and other homeotherms (for example, mammals (e.g., rabbit, sheep, goat, rat, mouse, guinea pig, bovine, horse and swine) and birds (e.g., domestic fowl, pigeon, duck, goose, quail, etc.)) and comprises the same or substantially the same amino acid sequences as the amino acid sequences represented by SEQ ID NO: 11 as GALR1, SEQ ID NO: 12 as GALR2 and SEQ ID NO: 13 as GALR3. In other words, the receptor protein includes a protein comprising an amino acid sequence having about 90 to 99.9% of homology with the amino acid SEQ ID NO: 11, 12 or 13 and having substantially the same quality of activity as the protein comprising the amino acid sequence represented by SEQ ID NO: 11, 12 or 13, in addition to the protein having the amino acid sequence represented by SEQ ID NO: 11, 12 or 13.

The activities manifested by these proteins include, for example, a ligand binding activity and signal transduction activity. The phrase "substantially the same quality of" mean that these properties are qualitatively the same. Accordingly, quantitative factors such as the strengths of the ligand binding activity and signal transduction activity and the molecular weights of the receptor proteins may be different among the receptor proteins.

The receptor protein also includes a protein of which N-terminal Met is protected with a protective group (for example, C₁₋₆ alkanoyl group such as formyl group or acetyl group), a protein of which N-terminal glutamine residue is converted into pyrroglutamic acid, a protein of which side chains of the intramolecular amino acids are protected with an appropriate protective group (for example, C₁₋₆ alkanoyl group such as formyl group or acetyl group) and a composite protein such as a so-called glycoprotein having sugar chains.

The same salts of the receptor protein as the salts of the peptide described above may be included in the present invention.

The receptor protein, a salt thereof or a partial peptide thereof may be produced from the tissues or cells of human and homeotherms by the protein purification methods known in the art, or by the same method of cultivation of a transformant comprising the DNAs encoding the peptide described above. The receptor protein may be also produced according to the peptide synthesis method as described above.

The partial peptide of the receptor protein include, for example, the moiety of the G-protein conjugate receptor protein exposed out of the cell membrane. This partial peptide may be a peptide containing a moiety identified to be the extracellular region (hydrophilic site) by a hydrophobicity plot analysis of the G-protein conjugate receptor protein, or a peptide containing a hydrophobic site as a part thereof. While a peptide comprising any one of independent domains may be used, it may contain a plurality of domains together.

The same peptide salt described above may be used as the salt of the partial peptide of the receptor protein.

Any DNA comprising a nucleotide sequence encoding the galanin receptor protein containing the same or substantially the same amino acid sequence as the amino acid sequence represented by SEQ ID NO: 11, 12 or 13 may serve as the DNA encoding the galanin receptor protein. The DNA thereof may be any one of the genome DNA, the genome DNA library, cDNA derived from the tissue or cells, cDNA library derived from the tissue or cells, and synthetic DNA. The vector to be used in the library may be any one of bacteriophage, plasmid, cosmid and phagemid. The DNA may be directly amplified by a RT-PCR method known in the art using a RNA fraction prepared from the tissue or cells.

The DNAs having the nucleotide sequence represented by SEQ ID NO: 17, 18 and 19, respectively, are used as the DNAs encoding the galanin receptor having the amino acid sequences represented by SEQ ID NO: 11, 12 and 13, respectively. SEQ ID NO: 17 represents the cDNA nucleotide sequence of the rat GALR1 (galanin receptor type 1), SEQ ID NO: 18 represents the cDNA nucleotide sequence of the rat GALR2 (galanin receptor type 2), and SEQ ID NO: 19 represents the cDNA nucleotide sequence of the rat GALR3 (galanin receptor type 3).

Since the GALR2 (galanin receptor type 2) is widely distributed in the hippocampus, hypothalamus, uterus, kidney, prostate and skeletal muscle, the peptide of the present invention which is an agonist having a GALR2 activation ability (or an antagonist against GALR2, or a neutralizing antibody against the peptide of the present invention) may be used as a memory function improving agent, an appetite improving agent or a function improving agent for uterus, kidney, prostate or skeletal muscle.

Since the GALR1 (galanin receptor type 1) is widely distributed in the hypothalamus, hippocampus andpancreas, the peptide of the present invention which is an agonist having a GALR1 activation ability (or an antagonist against GALR1 or a neutralizing antibody against the peptide of the present invention) may be used as an anti-obesity agent, intelligence-tropic drug and insulin secreting drug.

Since the GALR3 (galanin receptor type 3) is widely distributed in the testis, spleen, heart, hypothalamus and pituitary gland, the peptide of the present invention which is an agonist having a GALR3 activation ability (or an antagonist against GALR3 or a neutralizing antibody against the peptide of the present invention) may be used as function improving agents for testis, spleen and heart.

The present invention provides (1) a method for screening a galanin receptor activating substance (an agonist) or a salt thereof, wherein a cell membrane fraction expressing a galanin receptor is prepared by cultivating a transformant (transformed cells) comprising a DNA that encodes the galanin receptor, and the binding amounts of ³⁵S-labeled guanosine-5'-0-3-thiotriphosphate to the cell membrane fraction are measured when a test substance is (a) in contact with and (b) in no contact with the cell membrane fraction, respectively, followed by comparing the values obtained with each other. The present invention also provides (2) a method for screening a galanin receptor activation inhibiting substance (an antagonist) or a salt thereof, wherein a cell membrane fraction expressing a galanin receptor is prepared by cultivating a transformant (transformed cells) comprising a DNA that encodes the galanin receptor, and the binding amounts of ³⁵S-labeled guanosine-5'-O-3-thiotriphosphate to the cell membrane fraction are measured when a galanin or the peptide of the present invention is (a) in contact with and (b) under presence of a test substance, in contact with the cell membrane fraction, respectively, followed by comparing the values obtained with each other.

The screening method according to the present invention will be described in detail hereinafter.

Although any galanin receptor protein may be used so long as the protein comprises any one of GALR1, GALR2 and GALR3 galanin receptor proteins or partial peptide thereof having its receptor function, preferred is the cell membrane fraction prepared from culture cells in which the galanin receptor protein is expressed in a large quantity by using a transformant (transformed cells) (preparation is described hereinafter).

The cells comprising the galanin receptor protein are host cells expressing the galanin receptor protein, and the host cells include, for example, yeast, insect cells and mammal cells, and the mammal cells are preferred.

The membrane fraction is a fraction containing many cell membranes obtained by a method known in the art, per se, after homogenizing the cells. Examples of the homogenizing method include a method for crushing the cells with a Potter-Elvehjem type homogenizer, homogenization with a Waring blender and Polytron (made by Kinematica Co.), homogenization by ultrasonic wave, and homogenization by spouting the cells out of fine nozzles while pressurizing the cells with a French press. Fractionation by centrifugal separation or by density gradient centrifugation is mainly used for fractionation of the cell membrane. For example, the cell homogenate is centrifuged at a low speed (500 to 3000 rpm) for a short period of time (usually about 1 to 10 minute), followed by a high speed centrifugation of supernatant (15000 to 30000 rpm) for 30 minutes to 2 hours to obtain a precipitation as a membrane fraction. The membrane fraction mainly comprises membrane proteins and phospholipids constituting the cell membrane including the expressed galanin receptor as well as G-proteins expressed by the cell itself.

The amount of the galanin receptor in the cells and membrane fraction comprising the galanin receptor is preferably 1 to 100 pmol, more preferably 5 to 20 pmol, per 1 mg of the membrane fraction protein. The larger is the amount of the expressed galanin receptor, the higher is the activity (ligand binding activity or specific activity) for activating the receptor per unit quantity of the membrane fraction, making it possible to construct a highly sensitive screening system while enabling a large quantity of the samples to be measured in one lot.

In the method for screening a compound (an agonist) for activating the galanin receptor, a standard sample of the receptor is prepared at first by dispersing the cell membrane fraction comprising the galanin receptor in a buffer solution suitable for screening. The buffer solution may be either a phosphate buffer or a Tris-HCl buffer containing about 1 to 5 mM of magnesium ions with a pH in the range of about 4 to 10 (preferably in the range of about 6 to 8) with optionally adding about 0.1 nM to 100 µM (preferably about 0.1 to 1 µM) of guanosine diphosphate. A protease inhibitor such as PMSF, leupeptin E-64 (made by Peptide Research Laboratories, Inc.) or pepstatin may be added in order to suppress the receptor and the test substance from being decomposed by a protease. A prescribed quantity (5000 cpm to 50000 cpm) of ³⁵S-labeled guanosine-5'-O-3-thiotriphosphate and the test substance are added in about 0.01 to 10 ml of the receptor solution. A reaction system (a reference group) is also prepared by adding only ³⁵S-labeled guanosine-5'-O-3-thiotriphosphate into the receptor solution without adding any test compounds. The reaction temperature and reaction time is about 0 to 50°C, preferably about 4 to 37°C, and about 20 minutes to 24 hours, preferably about 30 minutes to 3 hours, respectively. After the reaction, the reaction solution is filtered with a glass fiber filter, the filtrate is washed with an appropriate volume of the same buffer as described above, and specific radioactivity of ³⁵S-labeled guanosine-5'-O-3-thiotriphosphate left behind on the glass fiber filter is measured with a liquid scintillation counter. When a compound exhibits a higher increment of radioactivity by adding the test compound than the increment of radioactivity obtained without adding any test compound, the compound can be selected as a candidate compound having an ability for activating the galanin receptor.

For screening a compound (an antagonist) that inhibits activation of the galanin receptor, cell membrane fractions are prepared by the same method as used in screening of the above-mentioned agonist, and a prescribed amount of ³⁵S-labeled guanosine-5'-O-3-thiotriphosphate (5000 cpm to 50000 cpm), 10⁻⁴ to 10⁻⁶ M of galanin or the peptide of the present invention, and a test substance are added to the cell membrane fraction. A reaction system (a reference group) is also prepared by adding ³⁵S-labeled guanosine-5'-O-3-thiotriphosphate and galanin or the peptide to the present invention without adding any test compound. The systems are subjected to the same reaction as described above, and the compound can be selected as a candidate compound having an ability for inhibiting activation of the galanin receptor when the compound exhibits a larger reduction of radioactivity by adding the test compound than the reduction of radioactivity obtained without adding any test compound.

The test substance may include, for example, a peptide, protein, non-peptide compound, synthetic compound, fermentation product, cell extract, plant extract and animal tissue extract that may be a novel compound or a compound known in the art.

Since the galanin receptor agonist has the same action as the action of the peptide of the present invention against the galanin receptor, the agonist is useful as safe and less toxic medicine as the peptide of the present invention.

On the contrary, since the galanin receptor agonist can suppress the physiological activity of the peptide according to the present invention against the galanin receptor protein, the agonist is also useful as a safe and less toxic medicine for suppressing the receptor activity.

The salts of the substance obtained by the screening method above include pharmaceutically acceptable salts, for example a salt of an inorganic base, a salt of an organic base, a salt of an inorganic acid, a salt of an organic acid, and a salt of a basic or acidic amino acid.

Examples of favorable salts with the inorganic bases include alkali metal salts such as sodium salts and potassium salts, alkali earth metal salts such as calcium salts and magnesium salts, and aluminum salts and ammonium salts.

Examples of favorable salts with the organic bases include salts of trimethylamine, triethylamine, pyridine, picoline, 2, 6-lutidine, ethanolamine, diethanolamine, triethanolamine, cyclohexylamine, dicyclohexylamine and N,N-dibenzyl ethylenediamine.

Examples of favorable salts with the inorganic acids include salts of hydrochloric acid, hydrobromic acid, sulfuric acid and phosphoric acid.

Examples of favorable salts with the organic acids include salts of formic acid, acetic acid, propionic acid, fumaric acid, oxalic acid, tartaric acid, maleic acid, citric acid, succinic acid, malic acid, methanesulfonic acid, benzenesulfonic acid and benzoic acid.

Examples of favorable salts with the basic amino acids include salts of arginine, lysine and ornithine, and examples of favorable salts with the acidic amino acids include salts of aspartic acid and glutamic acid.

The substance obtained by the screening method of the present invention or the salts thereof, may be used as a medicine as described above by the same way as the peptide of the present invention as a medicine.

Antibodies (e.g., polyclonal antibody, monoclonal antibody) or antiserum against the peptide of the present invention may be produced by the method for producing the antibody or antiserum known in the art using the peptide of the present invention as an antigen.

For example, the polyclonal antibody may be produced according to the method described hereinafter.

### [Production of polyclonal antibody]

The polyclonal antibody against the peptide of the present invention may be produced according to the method known in the art, per se. For example, a complex of an immunological antigen (a peptide antigen) with a carrier protein is prepared; a homeotherm (for example a mammal such as rabbit, sheep, goat, rat, mouse, guinea pig, bovine, horse and swine) or a bird (for example fowl, pigeon, duck, goose and quail) is immunized by the same method for producing a monoclonal antibody bellow-mentioned, and a fraction containing an antibody against the peptide of the present invention is sampled from the immunized animal followed by separation and purification.

With respect to the complex of an immunological antigen with a carrier protein used in immunizing a mammal, any kind of the carrier protein may be cross-linked with the hapten (the peptide of the present invention or a partial peptide thereof) to be used for immunizing the mammal in any ratio, so long as the antibody is efficiently produced by immunizing with the hapten cross-linked to the carrier. For example, about 0.1 to 20 parts by weight, preferably about 1 to 5 parts by weight of the carrier protein such as bovine serum albumin, bovine cycloglobulin or keyhole limpet hemocyanin is coupled with one parts by weight of the hapten.

Various condensing agents can be used for the coupling of the hapten and carrier, and they include reagents such as glutaraldehyde, carbodiimide, a maleimide active ester and an active ester having a thiol group or dithiopyridyl group.

The condensation product, per se or together with a carrier and diluent, is administered to an antibody producing site of thewarm-blooded animal described above. In the administration, a Freund's complete adjuvant or a Freund's incomplete adjuvant may be administered for enhancing antibody production ability. The antigen is usually administered once per two to six weeks with three to ten challenges in total.

The polyclonal antibody is collected from the blood or ascites, preferably from the blood, of the mammal immunized as described above.

The antibody titer against the peptide of the present invention in the antiserum may be determined by the same method as measuring of the antibody titer of the cultivation supernatant of a hybridoma bellow-mentioned. The antibody may be separated and purified according to the same separation and purification method of immunoglobulin as that of the monoclonal antibody described below.

The monoclonal antibody can be produced by the following method.

### [Production of monoclonal antibody]

### (a) Preparation of monoclonal antibody producing cell

The peptide of the present invention is administered, per se or together with a carrier and diluent, to an antigen producing site of a warm-blooded animal (for example, a mammal (e.g., rabbit, sheep, goat, rat, mouse, guinea pig, bovine, horse and swine) and a bird (e.g., domestic fowl, pigeon, duck, goose and quail)). In the administration, a Freund's complete adjutant or Freund's incomplete adjuvant may be administered for enhancing antibody producing ability. The antigen is administered one per two to six weeks with two to ten challenges in total.

For preparing monoclonal antibody producing cells, individual animals exhibiting an antibody titer are selected from the warm-blooded animal, for example mouse, immunized with the antigen, spleen or lymph node is sampled two to five days after the final challenge, and a monoclonal antibody producing hybridoma is prepared by fusing the antibody producing cells contained in the spleen or lymph node to myeloma cells. The antibody titer in the antiserum is determined by measuring the activity of a marker bound to the antibody, after allowing the peptide, or a partial peptide thereof, labeled with a marker to be described hereinafter to react with the antiserum. The cells may be fused according to known methods such as the method by Keller and Milstein (Nature, 256, 495 (1975)). Polyethylene glycol (PEG) and Sendai virus, preferably PEG, may be used as a fusion enhancer.

While examples of the myeloma cell lines include NS-1, P3U1, SP2/0 and AP-1, the P3U1 cell line is preferably used. The ratio of the number of the antibody producing cells (spleen cells) to that of myeloma cells is preferably in the range of 1:1 to 20:1, PEG (preferably PEG 1000 to PEG 6000) is added in a concentration range of 10 to 80%, and the cells are efficiently fused by incubating at 20 to 40°C, preferably at 30 to 37°C, for one to ten minutes.

The antibody producing hybridoma against the peptide of the present invention may be screened by various methods. For example, the supernatant of the hybridoma culture is added to a solid phase (for example, a microplate) on which the peptide antigen of the present invention is adsorbed directly or together with the carrier; an anti-immunoglobulin antibody labeled with a radioactive marker or an enzyme marker (an anti-mouse immunoglobulin antibody is used when the cells used for cell fusion are derived from mouse) or protein A is added to the solid phase; and the monoclonal antibody against the peptide of the present invention bound to the solid phase is detected. Alternatively, the hybridoma culture supernatant is added to the solid phase on which the anti-immunoglobulin antibody or protein A is adsorbed; the peptide of the present invention labeled with a radioactive marker or an enzyme marker is added to the solid phase; and the monoclonal antibody against the peptide of the present invention bound to the solid phase is detected.

The monoclonal antibody against the peptide of the present invention may be selected by a method known in the art, per se, or a modified method thereof. The monoclonal antibody is usually selected in an animal cell culture medium supplemented with HAT (hypoxanthine, aminopterin and thymidine). Any culture medium may be used for the selection and breeding medium, so long as the hybridoma is able to grow in the medium. For example, the culture medium available is a RPM1 1640 medium containing 1 to 20%, preferably 10 to 20%, of fetal bovine serum, GIT culture medium containing 1 to 10%, of fetal bovine serum (made by Wako Pure Chemicals, Co., Inc.), or a serum free culture medium for cultivation of the hybridoma (SFC-101, made by Nissui Pharmaceuticals, Co.). The cultivation temperature is usually 20 to 40°C, preferably about 37°C, while the cultivation time is usually five days to three weeks, preferably one week to two weeks. The cells are usually cultivated under a 5% carbon dioxide atmosphere. The antibody titer of the supernatant of the hybridoma culture may be measured by the same method as the measuring of the antibody titer against the peptide of the present invention in the antiserum.

### (b) Purification of monoclonal antibody

The monoclonal antibody against the peptide of the present invention is separated and purified by the same separation and purification method of the immunoglobulin as used in separation and purification of the usual polyclonal antibody, wherein only the antibody is collected by a combination of separation methods such as, for example, salting-out, alcohol precipitation, isoelectric precipitation, electrophoresis, adsorption and desorption with an ion-exchange resin (such as DEAE), ultra-centrifugation and gel filtration, and a specific purification method using an active adsorbent such as a antigen-bound solid support, protein A or Protein G, followed by dissociation of binding to obtain the antibody.

Since the antibody against the peptide of the present invention produced according to the methods (a) and (b) above can specifically recognize the peptide of the present invention, the antibody may be used for determining the peptide of the present invention in the test solution, in particular for determining by a sandwich immunoassay. That is, the present invention provides, for example:
(i) a quantitative assay method of the peptide of the present invention in the test solution comprising allowing the antibody which reacts with the peptide of the present invention, to competitively react with the test solution and the labeled peptide of the present invention, and measuring the proportion of the labeled peptide of the present invention that has been bound to the antibody, and
(ii) a quantitative assay method of the peptide of the present invention in the test solution comprising allowing the test solution to react with an antibody insolubilized on a carrier and a labeled antibody simultaneously or continuously and then measuring the activity of the marker on the insolubilized carrier, wherein one of the antibody recognizes the N-terminal of the peptide of the present invention while the other antibody reacts with a site other than the N-terminal of the peptide of the present invention (for example the C-terminal).

While the peptide of the present invention may be measured using a monoclonal antibody that recognizes the peptide of the present invention, detection of the peptide is also possible using tissue staining. Either the antibody itself, or a F(ab')₂, Fab' or Fab fraction of the antibody may be used for detecting the peptide. The measuring method using the antibody of the present invention is not particularly restricted, and any methods are available, so long as the amount of antibody, antigen or antibody-antigen complex corresponding to the amount of the antigen (for example the amount of the ligand peptide) in the test solution is detected by chemical or physical means, and is calculated based on a standard calibration curve prepared using a standard solution containing a known amount of the antigen. While a nephelometric, competitive, immunometric and sandwich methods are favorably used, the sandwich method to be described hereinafter is particularly preferable in its sensitivity and specificity.

The marker to be used in the method for measurement using the marker include radioactive isotopes, enzymes, fluorescent substances and luminescent substances. Examples of the radioactive isotopes include [¹²⁵I], [¹³¹I], [³H] and [¹⁴C]. As the enzymes above, a stable enzyme having large specific activity is preferred such as, for example, β-galactosidase, β-glucosidase, alkali phosphatase, peroxidase and malic acid dehydrogenase. Examples of the fluorescent substance include fluorescamine and fluorescein isothiocyanate, while examples of the luminescent substance include luminol, luminol derivatives, luciferin and lucigenin. A biotin-avidin complex may be used for binding the antibody or antigen with a marker.

The antigen or antibody may be insolubilized by either physical adsorption, or by a chemical bond that is usually used for insolublizing or immobilizing a protein or an enzyme. Examples of the carrier include insoluble polysaccharides such as agarose, dextran and cellulose, synthetic resins such as polystyrene, polyacrylamide and silicone, and a glass.

In the sandwich method, the amount of the peptide of the present invention in the test solution can be determined by allowing an insolubilized antibody against the peptide of the present invention to react with the test solution (primary reaction), further allowing the labeled antibody against the peptide of the present invention to react with the reaction product in the primary reaction (secondary reaction), and measuring the activity of the insolubilized marker on thecarrier. The order of the primary and secondary reactions may be reversed, or both reactions may be carried out simultaneously or with a time interval.

The same methods as described above may be used for labeling and insolubilizing. The antibodies to be used for the immobilized or labeled antibodies is not always required to be one kind, and a mixture of at least two kinds of the antibodies may be used for improving sensitivity.

In the method for measuring the peptide of the present invention by the sandwich method of the present invention, the antibodies having different binding sites against the peptide of the present invention is preferred as the antibodies against the peptide of the present invention used in the primary and secondary reactions respectively. For example, when the antibody to be used in the secondary reaction recognizes the C-terminal of the peptide of the present invention, the antibody to be used in the primary reaction preferably recognizes the site other than the C-terminal, for example the N-terminal.

The antibody against the peptide of the present invention may be used in other measuring systems other than the sandwich method, for example for the competitive method, immunometric method and nephelometry. In the competitive method, the antigen in the test solution and the labeled antigen are allowed to competitively react with the antibody, unreacted labeled antigen (F) and labeled antigen (B) bound to the antibody are separated with each other (B/F separation), and the amount of labeling of either B or F is measured to determine the amount of antigen in the test solution. As these reaction methods, used is a liquid phase method in which a soluble antibody is used as the antibody and polyethylene glycol or a second antibody against the antibody above is used for B/F separation and a solid phase method in which the immobilized antibody is used as the first antibody or a soluble antibody is used as the first antibody and an immobilized antibody is used as the second antibody.

In the immunometric method, the antigen in the test solution and immobilized antigen are allowed to competitively react with a prescribed amount of the labeled antibody followed by separation of the solid phase from the liquid phase; or the antigen in the test solution is allowed to react with an excess amount of the labeled antibody, and the immobilized antigen is added to allow the unreacted labeled antibody to bind to the solid phase followed by separation of the solid phase from the liquid phase. Subsequently, the amount of labeling in either of the two phases is measured to determine the amount of the antigen in the test solution.

In the nephelometry, the amount of insoluble precipitates generated as a result of the antigen-antibody reaction in the solution or in the gel is measured. Laser nephelometry taking advantage of laser scattering may be favorably applied even when only a small amount of the precipitate is obtained due to a minute content of the antigen in the test solution.

Setting of any special conditions and operations is not needed for applying these immunological measuring methods to the measuring method of the present invention. A measuring system for the peptide of the present invention or a partial peptide thereof may be constructed in addition to technical considerations common to those skilled in the art in the usual conditions and operation methods in each method. Details of general technical means are referenced in the following summaries and publications: Hiroshi Irie, ed., Radio Immunoassay, Kodansha, 1974; Hiroshi Irie, ed., Radio Immunoassay, Second Volume, Kodansha, 1979; Eiji Ishikawa et. al., ed., Enzyme Immunoassay, Igaku Shoin, 1978; Eiji Ishikawa et. al., ed., Enzyme Immunoassay, Second Edition, Igaku Shoin, 1982; Eiji Ishikawa et. al., ed., Enzyme Immunoassay, Third Edition, Igaku Shoin, 1987; Method in Enzymology, Vol. 70, Immunochemical Techniques Part A;ibid., Vol. 73, Immunochemical Techniques Part B;ibid., Vol. 74, Immunochemical Techniques Part C;ibid., Vol. 84, Immunochemical Techniques Part D, Selected Immunoassays;ibid., Vol. 92, Immunochemical Techniques Part E, Monoclonal Antibodies and General Immunoassay Methods;ibid., Vol. 121, Immunochemical Techniques Part I, Hybridoma Technology and Monoclonal Antibodies; all of which are published from Academic Press., Co.

Sensitive quantitative determination of the peptide of the present invention is possible by using the antibody against the peptide of the present invention as described above.

The present invention also provides a diagnostics by which the concentration of the peptide of the present invention is quantitatively determined using the antibody of the present invention. That is,
(1) When changes of the concentration of the peptide of the present invention have been detected, the patient may be diagnosed to have a disease such as obesity, dementia, diabetes or pituitary gland tumor, or have a high risk to fall in these diseases in the future.

The antibody of the present invention may be used for detecting the peptide of the present invention present in the test samples such as a body fluid or tissue. The antibody may be also used for preparing an antibody column for purification of the peptide of the present invention, for detecting the peptide of the present invention in each fraction during purification, and for analyzing the behavior of the peptide of the present invention in the test cells.

A transgenic animal expressing the peptide of the present invention can be created using the DNA of the present invention. Examples of the animal include mammals (e.g., rat, mouse, sheep, swine, bovine, cat, dog and monkey), particularly preferably mouse and rat. These animals are quite useful for analyzing the function of the peptide of the present invention, and for studies of diseases related to the peptide.

For transfer of the DNA of the present invention into the test animal, it is generally advantageous to use the DNA as a gene construct by binding the DNA to a downstream of a promoter capable of expressing the DNA in animal cells. When the DNA of the present invention derived from, for example, a mouse is transferred, a gene construct is created by conjugating the DNA of the present invention, which is derived from an animal highly analogous to the mouse, to the downstream of various promoters that are expressed in the animal cells. A DNA transfer animal having high productivity of the receptor protein of the present invention may be created by microinjection of the gene construct into a fertilized egg of, for example, the mouse. While a promoter derived from a virus or a ubiquitous expression promoter such as metallothionein may be used, a NGF gene promoter and enolase gene promoter that are specifically expressed in the brain are preferably used.

The DNA of the present invention is transferred in the fertilized egg cell stage so that the DNA is certainly distributed in all the germ cells and somatic cells. The presence of the peptide of the present invention in the germ cells of a created animal after the DNA transfer means that all the germ cells and somatic cells of the descendant of the created animal contain the peptide of the present invention. The peptide of the present invention is involved in all the germ cells and somatic cells in the descendant of the animal inheriting the gene.

The DNA transfer animal of the present invention, confirmed stably having the gene by mating, can be bred from generation to generation under a normal breeding environment as a DNA retaining animal. Furthermore, a homozygous animal having the introduced gene in both homologous chromosomes is obtained by crossbreeding male and female animals having the desired DNA, and descendants of the animal may be bred generation to generation by crossbreeding of the male and female animals so that the descendants also possess the desired DNA.

Since the peptide of the present invention is highly expressed in the animal having the transferred DNA of the present invention, the animal is useful for screening the agonist or antagonist against the peptide of the present invention.

The DNA transfer animal of the present invention can be used for a tissue culture cell source. For example, the peptide of the present invention may be analyzed by directly analyzing the DNA or RNA in the tissue of the transfer mouse of the present invention, or by analyzing the tissue containing the peptide of the present invention expressed by the gene. For example, the function of the cells derived from tissues such as brain and peripheral tissues that have been considered to be difficult to cultivate may be studied using the cells of the tissue comprising the peptide of the present invention cultivated by a standard tissue culture technology. By using these cells, it is also possible to select a medicine that can enhance the function of each tissue. And the peptide of the present invention may be also isolated and purified from the high expression cell lines.

A knock-out animal in which the function of the gene encoding the peptide of the present invention becomes deficient by destroying the gene may be created by the same method as described above. Such knock-out animal is also useful for analyzing the function of the peptide of the present invention.

The abbreviations of the base and amino acid in the specification and drawings are based on the abbreviation in IUPAC-IUB Commission on Biochemical Nomenclature, or on the commonly used abbreviation in the art. The amino acid means a L-isomer thereof unless otherwise stated, when the amino acid comprises optical isomers.

Examples thereof are as follows:
- DNA :: deoxyribonucleic acid
- cDNA :: complementary deoxyribonucleic acid
- A :: adenine
- T :: thymine
- G :: guanine
- C :: cytosine
- Y :: thymine or cytosine
- N :: thymine, cytosine, adenine or guanine
- R :: adenine or guanine
- M :: cytosine or adenine
- W :: thymine or adenine
- S :: cytosine or guanine
- I :: inosine
- H :: adenine, thymine or cytosine
- D :: guanine, adenine or thymine
- B :: guanine, thymine or cytosine
- RNA :: ribonucleic acid
- mRNA :: messenger ribonucleic acid
- dATP :: deoxyadenosine triphosphate
- dTTP :: deoxythymidine triphosphate
- dGTP :: deoxyguanosine triphosphate
- dCTP :: deoxycytidine triphosphate
- ATP :: adenosine triphosphate
- EDTA :: ethylenediamine tetraacetic acid
- SDS :: sodium dodecylsulfate
- EIA :: enzyme immunoassay
- Gly or G:: glycine
- Ala or A:: alanine
- Val or V:: valine
- Leu or L:: leucine
- Ile or I:: isoleucine
- Ser or S:: serine
- Thr or T:: threonine
- Cys or C:: cysteine
- Met or M:: methionine
- Glu or E:: glutamic acid
- Asp or D:: aspartic acid
- Lys or K:: lysine
- Arg or R:: arginine
- His or H:: histidine
- Phe or F:: phenylalanine
- Tyr or Y:: tyrosine
- Trp or W:: tryptophane
- Pro or P:: proline
- Asn or N:: asparagine
- Gln or Q:: glutamine
- pGlu :: pyrroglutamic acid
- Me :: methyl group
- Et :: ethyl group
- Bu :: butyl group
- Ph :: phenyl group
- TC :: tyazolidine-4(R)-carboxaimide

The substituents, protective groups and reagents often used in the specification are described by the following symbols:
- Tos :: p-toluenesulfonyl
- HONB :: N-hydroxy-5-norbornene-2,3-dicarboxyimide
- Bzl :: benzyl
- Cl₂-Bzl :: chlorobenzyl
- Z :: benzyloxycarbonyl
- Br-Z :: 2-bromobenzyloxycarbonyl
- Cl-Z :: 2-chlorobenzyloxycarbonyl
- Boc :: t-butyloxycarbonyl
- HOBt :: 1-hydroxybenztriazole
- DCC :: N,N'-dicyclohexylcarbodiimide
- TFA :: trifluoroacetic acid
- Fmoc :: N-9-fluolenylmethoxycarbonyl
- DNP :: dinitrophenyl
- Bum :: tertiary butoxymethyl
- Trt :: trityl
- PAM :: phenylacetamidemethyl
- BHA :: benzhydrilamine
- Bom :: benzyloxymethyl
- OcHex :: cyclohexyl ester
- MeBzl :: 4-methylbenzyl
- CHO :: formyl
- NMP :: N-methylpyrrolidone

The sequence table according to the present invention denotes the following sequences:
[SEQ ID NO: 1] amino acid sequence of mouse matured GALR2 ligand according to the present invention
[SEQ ID NO: 2] amino acid sequence of mouse GALR2 ligand precursor according to the present invention
[SEQ ID NO: 3] nucleotide sequence of cDNA of mouse GALR2 ligand according to the present invention
[SEQ ID NO: 4] nucleotide sequence of primer used for PCR for cloning cDNA of mouse GALR2 ligand
[SEQ ID NO: 5] nucleotide sequence of primer used in PCR for cloning cDNA of mouse GALR2 ligand
[SEQ ID NO: 6] nucleotide sequence of cDNA cloned using the primers of SEQ ID NO: 4 and 5
[SEQ ID NO: 7] nucleotide sequence of primer used for 5'-RACE for cloning cDNA of mouse type GALR2 ligand
[SEQ ID NO: 8] nucleotide sequence of primer used for 3'-RACE for cloning cDNA of mouse GALR2 ligand
[SEQ ID NO: 9] nucleotide sequence of primer used for PCR for cloning cDNA of mouse GALR2 ligand
[SEQ ID NO: 10] nucleotide sequence of primer used for PCR for cloning cDNA of mouse GALR2 ligand
[SEQ ID NO: 11] total amino acid sequence of rat GALR1 (galanin receptor type 1)
[SEQ ID NO: 12] total amino acid sequence of rat GALR2 (galanin receptor type 2)
[SEQ ID NO: 13] total amino acid sequence of rat GALR3 (galanin receptor type 3)
[SEQ ID NO: 14] amino acid sequence (described in WO 99/48920) of swine galanin receptor ligand
[SEQ ID NO: 15] amino acid sequence (described in WO 99/48920) of rat galanin receptor ligand
[SEQ ID NO: 16] amino acid sequence (described in WO 99/48920) of human galanin receptor ligand
[SEQ ID NO: 17] nucleotide sequence of cDNA of rat GALR1 (galanin receptor type 1)
[SEQ ID NO: 18] nucleotide sequence of cDNA of rat GALR2 (galanin receptor type 2)
[SEQ ID NO: 19] nucleotide sequence of cDNA of rat GALR3 (galanin receptor type 3)
[SEQ ID NO: 20] nucleotide sequence of cDNA of mouse GALR2 ligand precursor according to the present invention

The transformant Escherichia coli TPO10/pGR2ML1 obtained in Example 1 below has been deposited with the Institute for fermentation, Osaka (IFO) with an accession number IFO 16361 since February 2, 2000, and with the National Institute of Advanced Industrial Science and Technology, International Patent Organism Depository (IPOD; chuo-6, Higashi 1-1-1, Tsukuba-shi, Ibaragi, Japan) with an accession number FERM BP-7092 since March 16, 2000.

While the present invention is described in more detail with reference to examples, these examples do not restrict the scope of the present invention in any sense.

### Example 1 Cloning of mouse ligand peptide cDNA

DNAs were amplified by PCR with primers F/R120 and R/R120 (described in WO 99/48920) using mouse testis Marathon-Ready cDNA (derived from BALB/c mouse, made by CLONTECH CO.) as a template.

F/R120: 5'-AGGCTGGACCCTCAATAGTGCTGGTTAC-3' (SEQ ID NO:
4)

R/R120: 5'-CCATCTATGGCCTTCCACAGGTCTAGGA-3' (SEQ ID NO:
5)

The PCR reaction solution was prepared by mixing 0.5 µl of ExTaq (made by Takara Shuzo Co.), 5 µl of attached 10x PCR buffer, 4 µl of 2.5 mM dNTP mixture, 0.5 µl each of the primers F/R120 and R/R120 (10 µM each), 1 µl of the template cDNA and 34.5 µl of distilled water. The reaction conditions were initial denaturation at 94°C for 30 seconds, 35 cycles of reactions at 94°C /30 seconds - 62°C /30 seconds - 72°C /60 seconds, and a final elongation reaction at 72°C for 10 minutes. The DNA fragment obtained was cloned according to the method described in the attached instruction manual using TOPO TA Cloning Kit (made by Invitrogen Co.). The sequence of the cloned DNA was deciphered using ABI377 DNA Sequencer, thereby obtaining the sequence in SEQ ID NO: 6.

AGGCTGGACC CTCAATAGTG CTGGTTACCT CCTGGGTCCT GTCCTCCCCG
TTTCCTCCAA 60
GGCCGACCAG GGCAGGAAGA GAGACTCAGC TCTTGAGATC CTAGACCTGT
GGAAGGCCAT 120
AGATGG 126
(SEQ ID NO: 6)

Primers 1F/M120 (SEQ ID NO: 7) and 1R/M120 (SEQ ID NO: 8) were prepared from the sequence obtained, and were used for the experiments of 5'-RACE and 3'-RACE described below.

1F/M120: 5'-TCCAAGGCCGACCAGGGCAGGAAGAGAG-3' (SEQ ID NO: 7)

1R/M120: 5′-GGTCTAGGATCTCAAGAGCTGAGTCTCT-3′ (SEQ ID NO: 8)

The PCR reaction solutions of 5'-RACE and 3'-RACE were prepared by mixing 0.5 µl of Taq (made by Takara Shuzo Co.), 5 µl of attached 10x PCR buffer (500 mM of KCl, 25 mM of MgCl₂, 100 mM of Tris-HCl, pH 8.3), 4 µl of 2.5 mM dNTP mixture, 3 µl of 25 mM MgCl₂, 1 µl of 10 µM of primer F/R120 (for 3'-RACE) or 10 µM of primer R/R120 (for 5'-RACE), 1 µl of 10 µM of primer AP1 (primer AP1 is attached to Marathon-Ready cDNA Kit made by CLONTECH CO.,) , 5 µl of template cDNA (made by CLONTECH Co., mouse testis Marathon-Ready cDNA) and 31 µl of distilled water. The reaction conditions were initial denaturation at 94°C for 60 seconds, five cycles of reactions at 94°C /30 seconds - 72°C /120 seconds, five cycles of reactions at 94°C /30 seconds - 70°C /120 seconds, 25 cycles of reactions at 94°C /30 seconds - 68°C /120 seconds, and a final elongation reaction at 68°C for 10 minutes.

Subsequently, the DNA was amplified by a nested PCR using the PCR reaction solution as a template. The reaction solution was prepared by mixing 0.5 µl of Taq (made by Takara Shuzo Co.), 5 µl of attached 10x PCR buffer (500 mM of KCl, 25 mM of MgCl₂, 100 mM of Tris-HCl, pH 8.3), 4 µl of 2.5 mM dNTP mixture, 3 µl of 25 mM of MgCl₂, 1 µl of 10 µM primer 1F/M120 (for 3'-RACE) or 10 µM primer 1R/M120 (for 5'-RACE), 1 µl of 10 µM promer AP2 (the primer AP2 is attached to Marathon-Ready cDNA kit made by CLONTECH Co.), 5 µl of template DNA (50 times of dilution of the PCR reaction solution) and 31 µl of distilled water. The reaction conditions were initial denaturation at 94°C for 60 seconds, five cycles of reactions at 94°C /30 seconds - 72°C /120 seconds, five cycles of reactions at 94°C /30 seconds - 70°C /120 seconds, 25 times of reactions at 94°C /30 seconds - 68°C /120 seconds, and final elongation reaction at 68°C for 10 minutes.

The DNA fragment obtained was cloned according to the instruction manual attached to TOPO TA Cloning Kit (made by Invitron Co.). The cloned DNA sequence was deciphered to obtain sequence information of the 5'-terminal and 3'-terminal. The primers 1F/M650 and 1R/M650 were prepared from this sequence information.

1F/H650: 5'-GAAAGACAGTCTCAAATCAAGGACCTGC-3' (SEQ ID NO: 9)

1R/H650: 5′-TGAGCTGGAAACGAAGGAGGAGTGAGGT-3′ (SEQ ID NO: 10)

A single strand DNA was synthesized from testis poly(A)+RNA derived from Balb/c mouse (made by NIPPON GENE Co.) by a conventional method using Superscript II reverse transcriptase (made by GIBCO/BRL Co.). The DNA was amplified by PCR using the primers F/M650 and R/M650 as templates of the DNA. The PCR reaction solution was prepared by mixing 1 µl of Pfu DNA polymerase (made by Stratagene Co.), 5 µl of attached 10x PCR buffer, 4 µl of 2.5 mM dNTP mixture, 2.5 µl each of 10 µM primers F/M650 and R/M650, 1 µl of template DNA and 34.5 µl of distilled water. The reaction conditions were initial denaturation at 94°C for 30 seconds, 30 cycles of reactions at 94°C /30 seconds - 67°C /30 seconds - 72°C /four minutes, and final elongation reaction at 72°C for 10 minutes. The DNA fragment obtained was cloned according to the attached instruction manual using pCRII Blunt TOPO vector (made by Invitrogen Co.). The sequence of the cloned DNA was sequenced by a conventional method, thereby obtaining pGR2ML1 having 180 bp of DNA fragment (SEQ ID NO: 3) encoding mouse GALR2 ligand total length peptide. Escherichia coli TOP10 transformed by the plasmid was named as TOP10/pGR2ML1 The amino acid sequence estimated from the nucleotide sequence of this 180 base pairs is shown in SEQ ID NO: 2. The matured portion thereof is shown in SEQ ID NO: 1. The amino acid sequence at the matured portion was compared with amino acid sequences of swine small intestine galanin (galanin), swine GalR2 ligand (P-GALR2L), rat GalR2 ligand (R-GALR2L) and human GalR2 ligand (H-GALR2L), and found that the amino acid sequence of the matured portion has a high homology with the swine GalR2 ligand (P-GALR2L), rat GalR2 ligand (R-GALR2L) and human GalR2 ligand (H-GALR2L) (see Fig. 1).

### Industrial Applicability

As hitherto described, the peptide according to the present invention, or the precursor, amide, ester or salt thereof, has an ability for activating the galanin receptor. Accordingly, the peptide according to the present invention may be used for developing a medicine such as a function controlling agent having little side effect for hypothalamus, pituitary gland, uterus, kidney, prostate or skeletal muscle.

It is also made possible to screen a compound that changes binding to the galanin receptor, by using the peptide according to the present invention, or a precursor, an amide, an ester or a salt thereof.

### Sequence Table Free Text

SEQ ID NO. 4: Designed oligonucleotide Primer to amplify mouse GALR2 ligand
SEQ ID NO. 5: Designed oligonucleotide Primer to amplify mouse GALR2 ligand
SEQ ID NO. 7: Designed oligonucleotide Primer for 5'-RACE to amplify mouse GALR2 ligand
SEQ ID NO. 8: Designed oligonucleotide Primer for 3'-RACE to amplify mouse GALR2 ligand
SEQ ID NO. 9: Designed oligonucleotide Primer to amplify mouse GALR2 ligand
SEQ ID NO. 10: Designed oligonucleotide Primer to amplify mouse GALR2 ligand

## Claims

1. A peptide comprising the same or substantially the same amino acid sequence as that represented by SEQ ID NO: 1, or a precursor, an amide an eater or a salt thereof.

2. The precursor according to Claim 1 comprising the same or substantially the same amino acid sequence as that represented by SEQ ID NO: 2.

3. A DNA comprising the DNA encoding the peptide or a precursor thereof according to Claim 1.

4. The DNA according to Claim 3 comprising a nucleotide sequence represented by SEQ ID NO: 3 or SEQ ID NO: 20.

5. A recombinant vector comprising the DNA according to Claim 3.

6. A transformant transformed by the recombinant vector according to Claim 5.

7. A method for producing the peptide, or a precursor, an amide, an ester or a salt thereof according to Claim 1, comprising cultivating the transformant according to Claim 6 to produce and accumulate the peptide, or a precursor, an amide, an ester or a salt thereof according to Claim 1.

8. An antibody against the peptide or a precursor thereof according to Claim 1.

9. A medicine comprising the antibody according to Claim 8.

10. A diagnostics comprising the antibody according to Claim 8.

11. A medicine comprising the peptide, or a precursor, an amide, an ester or a salt thereof according to Claim 1.

12. The medicine according to Claim 11 which is a memory function improving agent, an appetite controlling agent, a uterus function controlling agent, a kidney function controlling agent, a prostate function controlling agent, a testis function controlling agent or a skeletal muscle function controlling agent.

13. A method for screening an agonist or antagonist against a receptor protein containing the same or substantially the same amino acid sequence as that represented by SEQ ID NO: 11, SEQ ID NO: 12 or SEQ ID NO: 13, which comprises using the peptide, or a precursor, an amide, an ester or a salt thereof according to Claim 1.

14. A compound or a salt thereof obtained by the screening method according to Claim 13.

15. A memory function improving agent, an appetite controlling agent, a uterus function controlling agent, a kidney function controlling agent, a prostate function controlling agent, a testis function controlling agent or a skeletal muscle function controlling agent, which comprises the compound or a salt thereof according to Claim 14.

16. Use of (1) the peptide, or a precursor, an amide, an ester or a salt thereof according to Claim 1, (2) the DNA according to Claim 3 or (3) the antibody according to Claim 8 for producing a medicine having a memory function improving action, an appetite controlling action, an uterus function controlling action, a kidney function controlling action, a prostate function controlling action, a testis function controlling action or a skeletal muscle function controlling action.

17. A method for improving memory function or for controlling appetite, uterus function, kidney function, prostate function, testis function or a skeletal muscle function, which comprises administering to mammal (1) the peptide, or a precursor, an amide, an ester or a salt thereof according to Claim 1, (2) the DNA according to Claim 3 or (3) the antibody according to Claim 8.
